(19) **Europäisches Patentamt European Patent Office Office européen des brevets**

(11) **EP 4 499 876 B1**

(12) **EUROPEAN PATENT SPECIFICATION**

(45) Date of publication and mention
of the grant of the patent:
**01.04.2026 Bulletin 2026/14**

(21) Application number: **23713888.8**

(22) Date of filing: **23.03.2023**

(51) International Patent Classification (IPC):
***C12Q 1/6886*** (2018.01)

(52) Cooperative Patent Classification (CPC):
**C12Q 1/6886;** C12Q 2600/106; C12Q 2600/156

(86) International application number:
**PCT/EP2023/057543**

(87) International publication number:
**WO 2023/180478 (28.09.2023 Gazette 2023/39)**

(54) **METHOD FOR DETECTING MICROSATELLITE INSTABILITY IN CANCER PATIENT**

VERFAHREN ZUM DETEKTIEREN VON MIKROSATELLITENINSTABILITÄT BEI EINEM KREBSPATIENTEN

PROCÉDÉ DE DÉTECTION D'INSTABILITÉ DE MICROSATELLITES CHEZ UN PATIENT CANCÉREUX

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **24.03.2022 EP 22305360**

(43) Date of publication of application:
**05.02.2025 Bulletin 2025/06**

(73) Proprietors:
• **Institut Curie**
 **75005 Paris (FR)**
• **INSERM (Institut National de la Santé et de la Recherche Médicale)**
 **75013 Paris (FR)**
• **Université de Versailles Saint-Quentin-en-Yvelines**
 **78000 Versailles (FR)**

(72) Inventors:
• **BIDARD, Francois-Clement**
 **75012 Paris (FR)**
• **KLOUCH, Khadidja**
 **78400 Chatou (FR)**
• **BORTOLINI SILVEIRA, Amanda**
 **55124 Mainz (DE)**
• **STERN, Marc-Henri**
 **75014 Paris (FR)**

• **RENAULT, Shufang**
 **92340 Bourg-la-Reine (FR)**
• **PROUDHON, Charlotte**
 **35000 Rennes (FR)**

(74) Representative: **Plasseraud IP**
 **104 Rue de Richelieu**
 **CS92104**
 **75080 Paris Cedex 02 (FR)**

(56) References cited:
**WO-A1-2021/130271**

• **EIRIKSDOTTIR G ET AL: "MAPPING OF CHROMOSOME 3 ALTERATIONS IN HUMAN BREAST CANCER USING MICROSATELLITE PCR MARKERS: CORRELATION WITH CLINICAL VARIABLES", INTERNATIONAL JOURNAL OF ONCOLOGY, DEMETRIOS A. SPANDIDOS ED. & PUB, GR, vol. 6, no. 2, 1 January 1995 (1995-01-01), pages 369 - 375, XP009024624, ISSN: 1019-6439**

Remarks:
The complete document including Reference Table(s) and the Sequence Listing(s) can be downloaded from the EPO website

Description

TECHNICAL FIELD

The present invention relates to a method for identifying a cancer patient presenting microsatellite unstable tumor who is likely to benefit from immunotherapy comprising detecting a mutation within a microsatellite sequence by subjecting said sample to a digital polymerase chain reaction. The present invention also encompasses an immune checkpoint inhibitor for use in the treatment of a cancer in a patient who is previously identified as likely benefit from an immunotherapy and a method for evaluating the therapeutic response of an immune checkpoint inhibitor in a patient having a cancer.

BACKGROUND OF THE INVENTION

Microsatellite instability-high (MSI-H) is a phenotype with hypermutability, resulting in tumor development through increasing cancer-related gene mutations (Lorenzi M, et al. J Oncol. 2020). This hypermutable phenotype mostly derives from a defective DNA mismatch repair (dMMR), which is responsible for recognizing and correcting misincorporations during DNA replication. A high concordance between dMMR deficiency and MSI status has been reported in different tumor types (Cicek MS et al. J Mol Diagnostics. 2011;13:271-81; McConechy MK, et al. Gynecol Oncol. 2015;137:306-10; Hause RJ, et al. Nat Med. 2016;22:1342-50). Originally, dMMR or MSI is sought in colorectal, endometrial and gastric cancers as a marker of Lynch syndrome, a hereditary cancer predisposition due to germline monoallelic deleterious mutations of an MMR gene, and to offer further genetic counselling. More recently, several clinical studies have demonstrated that dMMR or MSI-H is a predictive factor of tumor response to immune checkpoint inhibitors (Brahmer J, et al. N Engl J Med. 2015;373:123-35; Balar A, et al. Lancet. 2017;389:67-76; Le DT, Durham JN, et al. Science. 2017;357:409-13). In 2017, FDA approval of pembrolizumab for the treatment of advanced MSI-H or dMMR solid tumors led to a growing interest in MSI detection across a broad cancer spectrum including those with low yet non-negligible MSI prevalence.

Currently, methods used for MSI detection in clinics include immunohistochemistry (IHC), PCR-based assays and next-generation sequencing (NGS). IHC allows to study the loss of MMR proteins (MLH1, MSH2, MSH6, PMS2). PCR-based assays followed by capillary electrophoresis detect shifts in allele size of targeted microsatellites. The most common PCR-based commercial kit, known as the "pentaplex assay", interrogates 5 mononucleotide microsatellites (BAT-25, BAT-26, NR-21, NR-24, and NR-27), chosen to optimize the detection of MSI in colorectal cancers (Suraweera N, et al. Gastroenterology. 2002;123:1804-11). More recently, NGS has allowed to interrogate dozens or hundreds of microsatellites in a single assay, allowing for a more thorough evaluation of the MSI-H status in tumor samples. WO 2021/130271 discloses a method for predicting the efficacy of a treatment with immunotherapy by identifying microsatellite unstable tumours using digital polymerase chain reaction (drop-off probes).

Eiriksdottir et al. (INTERNATIONAL JOURNAL OF ONCOLOGY, 1995) discloses MSI on several chromosomes including chromosome 3 associated with breast cancer.

Breast cancer (BC) is the most commonly occurring cancer in women worldwide with over 2 million new cases in 2018. The prevalence of MSI-H among BC has been investigated in many reports by the above-mentioned techniques and is estimated of around 0.5-1.7 % (Willis J, Lefterova MI, et al. Clin Cancer Res. 2019;25:7035-45; Bonneville R, et al. JCO Precis Oncol. 2017;1-15; Cortes-Ciriano I, et al. Nat Commun; 2017;8:1-12). Because of its rare prevalence and the lack of evidence supporting the routine use of NGS in BC (Mosele F, et al. Ann Oncol. 2020;31:1491-505), MSI detection remains mostly unaddressed in BC patients, thus limiting their access to immune therapy.

SUMMARY OF THE INVENTION

In the present application, using publicly available whole-genome sequencing (WGS) data, the inventors identified a combination of 4 microsatellites often unstable in MSI-H breast tumors. Drop-off droplet-based digital PCR assays targeting these microsatellites were then developed and validated. Using a large collection of breast cancer samples, the inventors validated the assay performances in tumor tissue DNA and cell-free circulating DNA (cfcDNA). The drop-off ddPCR assays allow for accurate determination of MSI status in tissue DNA and cfcDNA of BC patients. This non-invasive approach is rapid, cost-effective, and compatible with a large pre-screening of BC patients that may benefit from immunotherapy.

The present invention relates to an *in vitro* method for identifying a cancer patient presenting microsatellite unstable tumors, who is likely to benefit from immunotherapy, said method comprising the steps of:

i) detecting a mutation within at least one microsatellite sequence in a plurality of nucleic acid molecules of a tumor DNA sample by subjecting said sample to a digital polymerase chain reaction (PCR), preferably a droplet digital PCR wherein said microsatellite sequence is selected from the group consisting of: M1 localized between positions 93963773 to 93963781 of chromosome 3 (GRch37.p13 : GCF_000001405.25, updated on 28/06/2013), M2 localized between positions 36126599 to 36126607 of chromosome 4 (GRch37.p13 : GCF_000001405.25, updated on 28/06/2013), M3 localized between positions 65403357 to 6543365 of chromosome 1 (GRch37.p13 : GCF_000001405.25, updated on 28/06/2013) and M4 localized between positions 178753846 to 1787538554 of chromosome 1 (GRch37.p13 : GCF_000001405.25, updated on 28/06/2013),

ii) determining a copy number of each microsatellite mutated sequence(s) in said tumor DNA sample,

wherein a higher copy number of at least one mutated microsatellite sequence(s) as compared to corresponding threshold value(s) is indicative that the patient is likely to benefit from immunotherapy.

The method according to the present invention can further comprises the steps of:

iii) determining a copy number of each corresponding wild-type microsatellite sequence(s) in said tumor DNA sample and, iv) calculating for each microsatellite sequence, a mutant allelic frequency (MAF) of said microsatellite sequence with the following formula: MAF = copy number of mutated microsatellite sequence / (copy number of wild-type microsatellite sequence + copy number of mutated microsatellite sequence), wherein a higher MAF of at least one microsatellite sequence(s) as compared to a corresponding threshold value is indicative that the patient is likely to benefit from the immunotherapy.

In a preferred embodiment, the mutation is detected within at least two microsatellite sequences selected from the group consisting of: M1, M2, M3 and M4, and wherein a higher copy number or MAF of at least two mutated microsatellite sequences as compared to corresponding threshold values is indicative that the patient is likely to benefit from the immunotherapy.

According to a particular embodiment, said mutation is a deletion, substitution and/or addition of at least one repeat unit, preferably a deletion of at least one repeat unit.

In a particular embodiment, said digital polymerase chain reaction according to the present method comprises subjecting the tumor DNA sample to:

- a pair of primers for amplifying a target sequence including a mutated or wild-type microsatellite sequence selected from the group consisting of M1, M2, M3 and M4,

- an oligonucleotide probe (MS) comprising a sequence complementary to the wild-type microsatellite sequence and,

- an oligonucleotide reference probe (REF) comprising a sequence complementary to a part of said target sequence located outside of said microsatellite sequence,

preferably wherein said oligonucleotide microsatellite probe (MS) and oligonucleotide reference probe (REF) are labeled with different fluorophores.

In a more particular embodiment, when said microsatellite sequence is M1, the pair of primers are SEQ ID NO: 1 and 2, the oligonucleotide microsatellite probe (MS) is SEQ ID NO: 3 and oligonucleotide reference probe (REF) is SEQ ID NO: 4, when said microsatellite sequence is M2, the pair of primers are SEQ ID NO: 5 and 6, the oligonucleotide microsatellite probe (MS) is SEQ ID NO: 7 and oligonucleotide reference probe (REF) is SEQ ID NO: 8, when said microsatellite sequence is M3, the pair of primers are SEQ ID NO: 9 and 10, the oligonucleotide microsatellite probe (MS) is SEQ ID NO: 11 and oligonucleotide reference probe (REF) is SEQ ID NO: 12 and/or when said microsatellite sequence is M4, the pair of primers are SEQ ID NO: 13 and 14, the oligonucleotide microsatellite probe (MS) is SEQ ID NO: 15 and oligonucleotide reference probe (REF) is SEQ ID NO: 16.

In a preferred embodiment, said tumor DNA sample is tumor tissue or cell-free circulating DNA in biological fluids, preferably in plasma.

According to the present invention, said cancer can be selected from the group consisting of lung cancer, prostate cancer, endometrial cancer, colorectal cancer, ovarian cancer, tumor of the Lynch syndrome, breast cancer and gastric cancer, preferably breast cancer.

In another aspect, the present invention relates to an immune checkpoint inhibitor for use in the treatment of a cancer in a patient in need thereof wherein said immune checkpoint inhibitor is administered in a patient who is previously identified as likely to benefit from an immunotherapy using the method as described above.

The present invention also relates to a method for evaluating the therapeutic response of an immune checkpoint inhibitor in a patient having a cancer, said method comprising: i) detecting a mutation within at least one microsatellite sequence in a plurality of nucleic acid molecules of a tumor DNA sample of a patient having received at least one dose of an immune checkpoint inhibitor, by subjecting said sample to a digital polymerase chain reaction, wherein said microsatellite sequence is selected from the group consisting of: M1, M2, M3 and M4, wherein a decrease of a copy number or MAF of at least one mutated microsatellite sequence in the patient sample during the treatment is indicative that the patient is responsive to the immune checkpoint treatment.

Finally, the present invention relates to a kit for identifying a mutation in a microsatellite sequence selected from the group consisting of: M1, M2, M3 and M4 from a DNA sample comprising:

- a pair of primers for amplifying a target sequence including a mutated or wild-type microsatellite sequence selected from the group consisting of M1, M2, M3 and M4,

- an oligonucleotide probe (MS) comprising a sequence complementary to the wild-type microsatellite sequence,

- an oligonucleotide reference probe (REF) comprising a sequence complementary to a part of said target sequence located outside of said microsatellite sequence, preferably,

- a pair of primers of SEQ ID NO: 1 and 2, the oligonucleotide microsatellite probe (MS) of SEQ ID NO: 3 and oligonucleotide reference probe (REF) of SEQ ID NO: 4,

- a pair of primers of SEQ ID NO: 5 and 6, the oligonucleotide microsatellite probe (MS) of SEQ ID NO: 7 and oligonucleotide reference probe (REF) of SEQ ID NO: 8,

- a pair of primers of SEQ ID NO: 9 and 10, the oligonucleotide microsatellite probe (MS) of SEQ ID NO: 11 and oligonucleotide reference probe (REF) of SEQ ID NO: 12 and/or

- a pair of primers of SEQ ID NO: 13 and 14, the oligonucleotide microsatellite probe (MS) of SEQ ID NO: 15 and oligonucleotide reference probe (REF) of SEQ ID NO: 16.

## DETAILED DESCRIPTION OF THE INVENTION

### Method for the detecting a mutation within at least one M1, M2, M3 or M4 sequence

In the present application, using publicly available whole-genome sequencing (WGS) data, the inventors identified four microsatellite sequences unstable in breast tumors:

- M1 localized between positions 93963773 to 93963781 of chromosome 3 (GRch37.p13 : GCF_000001405.25, updated on 28/06/2013),
- M2 localized between positions 36126599 to 36126607 of chromosome 4 (GRch37.p13 : GCF_000001405.25, updated on 28/06/2013),
- M3 localized between positions 65403357 to 6543365 of chromosome 1 (GRch37.p13 : GCF_000001405.25, updated on 28/06/2013) and
- M4 localized between positions 178753846 to 1787538554 of chromosome 1 (GRch37.p13 : GCF_000001405.25, updated on 28/06/2013).

The microsatellite sequences are identified by an identification number of the chromosomic coordinates using the reference genome GRCh37.p13 (Genome Reference Consortium Human Build 37 submitted on 28 June, 2013) (GenBank assembly accession: GCA_000001405.25).

Thus, the present disclosure relates to a method for detecting a mutation in at least one, preferably two, three or four microsatellite sequence(s) selected from the group consisting of M1, M2, M3 and M4.

As used herein, "microsatellite sequence" refers to a region of genomic DNA that contains short, repetitive sequence elements of one to seven, typically one to five, notably one to four base pairs in length. Each sequence repeated at least once within a microsatellite locus is referred to herein as a "repeat unit". Each microsatellite locus typically includes at least seven repeat units, notably at least ten repeat units, and preferably at least twenty repeat units, preferably includes at least nine repeat units.

Mutations at microsatellite sequence (also refers as microsatellite instability (MSI) typically include deletion(s), addition(s) or substitution of at least one repeat unit at a microsatellite locus. Typically, microsatellite instability results in a change in length at a microsatellite locus, due to addition(s) or most frequently deletion(s).

A mutation in at least one microsatellite sequence as described above can be detected by any methods known in the art including as non-limiting examples: immunohistochemistry (for example indirectly immunohistochemistry), DNA sequencing, PCR based method such as qPCR, digital PCR or ICE-cold PCR.

In particular embodiment, the mutated microsatellite sequence is detected by subjecting the sample (e.g., tumor DNA sample) to a PCR-based method (e.g., qPCR, or digital PCR) to:

- a pair of primers for amplifying a target sequence including a mutated or wild-type microsatellite sequence selected from the group consisting of M1, M2, M3 and M4,

- an oligonucleotide microsatellite probe (MS) comprising a sequence complementary to the wild-type microsatellite sequence and,

- an oligonucleotide reference probe (REF) comprising a sequence complementary to a part of said target sequence located outside of said microsatellite sequence.

"Target DNA sequence", or a "target (DNA) region" or a "target sequence" used interchangeably herein relates to the fragment of the DNA sample that is amplified by a pair of primers to form an amplicon. Typically, the amplicon is produced by Polymerase chain reaction (PCR). According to the invention, such target DNA sequence includes wild-type microsatellite sequence selected from the group consisting of M1, M2, M3 or M4 or a mutated microsatellite sequence comprising a mutation (e.g., includes deletion(s), addition(s) or substitution of at least one repeat unit) at said microsatellite locus.

As used herein, a "primer pair" refers to a pair of oligonucleotide primers that each hybridizes to a specific target nucleotide sequence, in particular a forward primer that hybridizes to a first location of a nucleic acid sequence; and a reverse primer that hybridizes to a second location of the nucleic acid sequence downstream of the first location that anneal to opposite strands of a nucleic acid sequence (typically including the microsatellite sequence) so as to form an amplicon specific to the target sequence during the PCR reaction.

Preferably, the primer pair is typically designed to have a melting temperature (Tm) lower than the critical denaturation temperature (Tc) of the reaction. The pair of primers can be designed using available computer programs.

Preferably primers are designed to generate amplicons of at least 80 base pairs (bp) notably at least 90 bp, or at least 100 bp. In some embodiments, primers are designed to generate amplicons under 150 bp, for compatibility with circulating DNA detection.

"Amplifying", as used herein, refers to a process whereby multiple copies are made of one particular locus of a nucleic acid (i.e., a target sequence as mentioned above), such as genomic DNA. Amplification is accomplished using PCR (Saiki et al., 1985 Science 230: 1350- 1354).

As used herein, an " oligonucleotide probe " (also named "probes", "hydrolysis probe", "TaqMan probe") refers to a probe that hybridizes to (i.e., which are complementary to) a specific target nucleotide sequence. Preferably probes are designed such that the melting temperature (Tm) of the probes is greater than the Tm of the primers. In some embodiments, the oligonucleotide probes according to the invention may include a minor groove binder (MGB) moiety at their 3' end. Such MGB typically increases the melting temperature (Tm) of the probe and stabilizes probe-target hybrids.

According to the present disclosure, a microsatellite oligonucleotide probe (MS) is a probe that hybridizes to (i.e., which are complementary to) a target sequence comprising at least a part of the wild-type microsatellite sequence as described above.

The probe size is designed to confer its ability to bind properly to the wild-type microsatellite sequence, while destabilizing hybridization of the MS probe in the presence of at least one mutation in the microsatellite sequence. In a particular embodiment, the oligonucleotide probes have a nucleotide sequence length of about 10 to about 50 nucleotides. Preferably, the oligonucleotide probes (and in particular the MS probe) have a nucleotide sequence length of at least 20 nucleotides, notably about 20 to 40, or 30 to 50 or notably 30 to 40 nucleotides.

In a preferred embodiment, MS probe covers the full wild-type microsatellite sequence and extends further a few nucleotides on each extremity (typically 1 to 10 nucleotides, notably 2 to 8, preferably 2 to 6, most preferably 2 to 5 or 2 to 4) to confer both its ability to bind properly and the resulting destabilization in case of mutation. In other words, the probe size is designed to confer its ability to bind properly to the wild-type microsatellite sequence, while preventing hybridization of the MS probes in the presence of a mutation in the microsatellite sequence.

According to the present disclosure, a reference oligonucleotide probe (REF) is a probe that hybridizes to (i.e., which are complementary to) a target sequence within the amplicon but at least partially located outside said microsatellite sequence. In a preferred embodiment, the REF probe is located outside the microsatellite. In another embodiment, the REF probe may partially overlap with said microsatellite sequence. In a preferred embodiment, the REF probe hybridizes with a sequence within the amplicon, which does not include microsatellite sequence M1, M2, M3 or M4. The REF probe is used to quantify the amplicon, comprising either a mutated or wild-type microsatellite sequence.

In a preferred embodiment, said probes are labelled, for example, with a fluorescent molecule. In a particular embodiment, said oligonucleotide microsatellite probe (MS) and oligonucleotide reference probe (REF) are labeled with different fluorophores to allow separate detection of their respective signal. In a preferred embodiment, the probes according to the present disclosure have a fluorophore covalently attached to their 5'-end of the oligonucleotide probe and a quencher.

According to the present disclosure, fluorescent molecule can be selected, for example, from the group consisting of FAM (5- or 6- carboxyfluorescein), VIC, NED, Fluorescein, FITC, IRD-700/800, CY3, CY5, CY3.5, CY5.5, HEX, TET (5-tetrachloro-fluorescein), TAMRA, JOE, ROX, BODIPY TMR, Oregon Green, Rhodamine Green, Rhodamine Red, Texas Red, Yakima Yellow, Alexa Fluor PET, Biosearch Blue™, Marina Blue®, Bothell Blue®, Alexa Fluor®, 350 FAM™, SYBR® Green 1, Fluorescein, EvaGreen™, Alexa Fluor® 488 JOE™, 25 VIC™, HEX™, TET™, CAL Fluor®Gold 540, Yakima Yellow®, ROX™, CAL Fluor® Red 610, Cy3.5™, Texas Red®, Alexa Fluor® 568 Cry5™, Quasar™ 670, LightCycler Red640®, Alexa Fluor 633 Quasar™ 705, LightCycler Red705®, Alexa Fluor® 680, SYT0®9, LC Green®, LC Green® Plus+, and FvaGreen™. Preferably, the detectable label is selected from 6-carboxyfluorescein, FAM, or tetrachlorofluorescein, (acronym: TET), Texas Red, Cyanin 5, Cyanine 3, or VIC™. Selection of adapted fluorophores is classical in the field can be typically achieved according to general recommendations. As a matter of example, well-suited fluorophores include FAM and VIC as used in the experimental results.

The quencher may be an internal quencher or a quencher located in the 3' end of the probe. Typical quenchers are tetramethylrhodamine, TAMRA, Black Hole Quencher or nonfluorescent quencher. Hydrolysis probes usable according to the invention are well-known in the field (see notably http://www.sigmaaldrich.com/technical-documents/articles/biology/quantitative-pcr-and-digital-pcr-detection-methods.html). The quencher molecule quenches the fluorescence emitted by the fluorophore when excited by the cycler's light source typically via FRET (Förster Resonance Energy Transfer). As long as the fluorophore and the quencher are in proximity, quenching inhibits any fluorescence signals. Such probes are designed such that they anneal within the target region amplified by a specific set of primers. As the Taq polymerase extends the primer and synthesizes the nascent strand, the 5'-3' exonuclease activity inherent in the Taq DNA polymerase then separates the 5' reporter from the 3' quencher, which provides a fluorescent signal that is proportional to the amplicon yield.

The fluorescence signal associated with the hybridization of the MS or REF probe can be measured by any method known in the art, for example with optical detector such as the Bio-Rad QX-100 droplet reader. In some embodiments, analysis may be achieved with appropriate software (such as the QuantaSoft v1.7.4 software for ddPCR or the ddPCR package on R [https://cran.r-project.org/web/packages/ddpcr/index.html]. Quantasoft allows manual assignment of the droplets to single REF positive or to the double REF/MS positive population (i.e., or clouds).

A threshold, under which a fluorescent signal is considered as "a residual fluorescent signal" can be determined by the one skilled in the art according to classical signal analysis techniques. Said threshold can be typically set using the R package that defines thresholds in an automatic way to avoid bias that might be introduced by manual assignment.

In a preferred embodiment, the pair of primers, oligonucleotide microsatellite probes and oligonucleotide reference probes that can be used to detect a mutation within M1, M2, M3 or M4 microsatellite sequences are listed in the Table 1 below:

**Table 1:** Examples of pair primers, oligonucleotide microsatellite probe (MS) and reference oligonucleotide probe (REF) that can be used to detect microsatellite (MS) sequence M1, M2, M3 and M4

| MS sequence | Forward primer | Reverse primer | MS probe | REF probe |
|---|---|---|---|---|
| M1 | AGTTCTCTGAATTTGCT CTGAATTA (SEQ ID NO : 1) | ACAAGTACATCCATAGA GTTCAAC (SEQ ID NO: 2) | TCTC**TTTTTTTT TCCC** (SEQ ID NO: 3) | CTGGAACTCTTAT TATCT (SEQ ID NO : 4) |
| M2 | TGCATTTGTTTGTGACT GTTTTAC (SEQ ID NO : 5) | TCTTTCCTTGTCATCTT GCGT (SEQ ID NO : 6) | AGATTG**TTTTTT TTT**CC (SEQ ID NO : 7) | CTGCTGATAGAC AAATCC (SEQ ID NO : 8) |
| M3 | CTCGCTTTAAGGATATG TCTG (SEQ ID NO : 9) | GTGGAACACTGATTAG TAACTG (SEQ ID NO : 10) | CAGG**TTTTTTTT T**GTC (SEQ ID NO : 11) | CTATGATCGACT AAGT (SEQ ID NO : 12) |
| M4 | TGTGGAGCAAACAGAG GGAT (SEQ ID NO : 13) | TTGCTACTTACCAAATT GAAGTGT (SEQ ID NO : 14) | CCCT**AAAAAAA AA**TGT (SEQ ID NO : 15) | CTTACTTTTCCCA CT (SEQ ID NO : 16) |

According to a particular embodiment, when microsatellite is M1, the pair of primers are SEQ ID NO: 1 and 2, the oligonucleotide microsatellite probe (MS) is SEQ ID NO: 3 and oligonucleotide reference probe (REF) is SEQ ID NO: 4.

According to another particular embodiment, when microsatellite is M2, the pair of primers are SEQ ID NO: 5 and 6, the oligonucleotide microsatellite probe (MS) is SEQ ID NO: 7 and oligonucleotide reference probe (REF) is SEQ ID NO: 8.

According to a particular embodiment, when microsatellite is M1, the pair of primers are SEQ ID NO: 9 and 10, the oligonucleotide microsatellite probe (MS) is SEQ ID NO: 11 and oligonucleotide reference probe (REF) is SEQ ID NO: 12.

According to a particular embodiment, when microsatellite is M4, the pair of primers are SEQ ID NO: 13 and 14, the oligonucleotide microsatellite probe (MS) is SEQ ID NO: 15 and oligonucleotide reference probe (REF) is SEQ ID NO: 16.

In a preferred embodiment, said oligonucleotide microsatellite probe (MS) and oligonucleotide reference probe (REF) as described above are labeled with different fluorophores.

The detection of the mutated microsatellite sequence as described above may be performed by submitting the DNA sample to a digital PCR technique. Typically, in such a technique, the PCR solution is divided in multiple compartments or droplets, which are made to run PCR individually. Typically, also most of the compartments or droplets contain either 0 or 1 copy of the target DNA fragment which is to be amplified.

According to the present disclosure, a mutation within at least one, preferably two, three or four microsatellite sequence(s) may be detected in a plurality of nucleic acid molecules of a DNA sample, preferably tumor DNA sample by subjecting said sample to a digital polymerase chain reaction (PCR).

As used herein, "digital PCR" refers to an assay that provides an end-point measurement that provides the ability to quantify nucleic acids without the use of standard curves, as is used in real-time PCR (see Sykes et al., 1992 Quantitation of targets for PCR by use of limiting dilution. BioTechniques 13,444-449, Vogelstein and Kinzler 1999 Digital PCR. Proc Natl Acad Sci USA, 96:9236-9241 and Pohl and Shihle 2004 Principle and applications of digital PCR. Expert Rev Mol Diagn, 4:41-47, see also Monya Baker 2012 Nature Methods 9, 541-544).

In a typical digital PCR experiment, a PCR solution is made similarly to a classical TaqMan probe assay, which typically comprises the DNA sample, probes (e.g., fluorescence-quencher probes), primers, and a PCR master mix, which generally contains DNA polymerase, dNTPs, $MgCl_2$, and reaction buffers at optimal concentrations. The PCR solution is then randomly distributed into partitions, such that some contain no nucleic acid molecule and others contain one or more nucleic acid molecule, preferably one nucleic acid molecule. Thus, in these conditions, the reference signal associated with the presence of the nucleic acid molecule in the DNA sample in a given compartment or droplet should be theoretically 0 or 1. Obviously due to biological variability for a population of partitions, clouds are observed corresponding respectively to the theoretic values 0 or 1.

The nucleic acid molecule contained in a partition is individually amplified to the terminal plateau phase of PCR (or end-point) and then the presence of the probes is detected (e.g. measure of fluorescence signal), to determine the fraction of positive compartments or droplets.

If the partitions are of uniform volume, the number of nucleic acid molecules present may be calculated from the fraction of positive partitions using Poisson statistics, according to the following equation:

$$\lambda = -\ln(1-p)$$

wherein $\lambda$ is the average number of nucleic acid molecules per replicate reaction and p is the fraction of positive end-point reactions. From $\lambda$, together with the volume of each replicate PCR and the total number of replicates analyzed, an estimate of the absolute target DNA concentration is calculated. Micro well plates, capillaries, oil emulsion, and arrays of miniaturized chambers with nucleic acid binding surfaces can be used to partition the samples in distinct compartments or droplets.

Techniques available for digital PCR include PCR amplification on a microfluidic chip (Warren et al., 2006 Transcription factor profiling in individual hematopoietic progenitors by digital RT-PCR. Proc Natl Acad Sci USA 103, 17807-17812; Ottesen et al., 2006 Microfluidic digital PCR enables multigene analysis of individual environmental bacteria. Science 314, 1464-1467; Fan and Quake 2007 Detection of aneuploidy with digital polymerase chain reaction. Anal Chem 79, 7576-7579). Other systems involve separation onto microarrays (Morrison et al., 2006 Nanoliter high-throughput quantitative PCR. Nucleic Acids Res 34, e123) or spinning microfluidic discs (Sundberg et al., 2010 Spinning disk platform for microfluidic digital polymerase chain reaction. Anal Chem 82, 1546-1550) and droplet techniques based on oil-water emulsions (Hindson, Benjamin et al., 2011 High-Throughput Droplet Digital PCR System for Absolute Quantitation of DNA Copy Number. Analytical Chemistry 83 (22): 8604-8610). Typically, digital PCR is selected from droplet digital PCR (ddPCR), BEAMing (beads, emulsion, amplification, and magnetic), and microfluidic chips. Preferably, the digital PCR is droplet digital PCR.

Typically, raw dPCR (or ddPCR) data are collected after PCR cycling by reading or measuring the fluorescence signal associated with the REF and MS probes for each compartment or droplet.

The PCR data collection step is typically performed in an optical detector (for example the Bio-Rad QX-100 droplet reader can be used in ddPCR). Preferably at least a two-color detection system is used (for example to detect FAM and either HEX or VIC fluorescent labels). Droplets clouds can typically be established on 2D graphs by plotting the fluorescence level for each probe per droplet. In some embodiments, analysis may be achieved with appropriate software (such as the QuantaSoft v1.7.4 software for ddPCR or the ddPCR package on R [https://cran.r-project.org/web/packages/ddpcr/index.html].

Quantasoft allows manual assignment of the droplets to the single REF positive or the double REF/MS positive population (i.e., or clouds). The R package defines thresholds in an automatic way to avoid bias that might be introduced by manual assignment.

The number of compartments or droplets that are positive for the reference probe (REF probe) can be used to quantify the total number of target sequence in the sample. The fraction of positive compartment or droplets can then be fitted to a Poisson distribution to determine the absolute initial copy number of the target sequence in the input reaction mixture in units of copies/$\mu$l.

In compartments or droplets containing a wild-type microsatellite sequence (no mutation in the targeted MS sequence), a maximum signal (e. g. fluorescent signal) is observed for both the REF and the MS probes. At the contrary, in compartments or droplets containing a mutated microsatellite sequence in the amplified target sequence a shift is observed for the signal (e. g. fluorescent signal) associated with the MS probe.

Most preferably, the digital PCR reaction is designed to ensure that most compartments (e.g. droplets) contain either 0 or 1 copy of targeted DNA fragment (notably depending on the quantity of DNA loaded in the reaction). In these conditions, an optimal separation of the WT (REF+/MS+ signals) vs. mutated microsatellite (or MSI) clouds (single REF+ signal) can be observed. It must be noted that due to biological variability that compartments or droplets classified in the single REF+ signal may include a residual (i.e., non-significant) MS signal. A threshold, under which a MS signal is considered as "a residual MS signal" can be determined by the one skilled in the art according to classical signal analysis techniques.

The number of copies of compartments or droplets containing wild type microsatellite sequences versus the number of copies of compartments or droplets containing mutated microsatellite sequences, may be used to determine the number of copies of the nucleic acid molecule of interest that were in the original sample and to calculate a mutant allelic frequency (MAF).

In a preferred embodiment, the method according to the present disclosure comprises the step of determining a copy number of corresponding wild-type microsatellite sequence in said tumor DNA sample and, calculating a mutant allelic frequency (MAF) of said microsatellite sequence with the following formula: MAF = copy number of mutated microsatellite sequence / (copy number of wild-type microsatellite sequence + copy number of mutated microsatellite sequence).

Typically, mutant allele frequency can be determined from compartment or droplet counts through manual assignment of WT and mutated microsatellite compartment or droplet clouds. As mentioned above, identification of a compartment or droplet population with a single signal from the REF probe indicates the presence of a mutated microsatellite sequence in the DNA sample.

In a preferred embodiment, said digital PCR is droplet digital PCR (ddPCR).

"Droplet digital PCR" (ddPCR) refers to a digital PCR assay that measures absolute quantities by counting nucleic acid molecules encapsulated in discrete, volumetrically defined, water-in-oil droplet partitions that support PCR amplification (Hinson et al., 2011, Anal. Chem. 83 :8604-8610; Pinheiro et al., 2012, Anal. Chem. 84: 1003-1011). A single ddPCR reaction may be comprised of at least 20,000 partitioned droplets per well.

A "droplet" refers to an individual partition of the PCR solution in a droplet digital PCR assay. A droplet supports PCR amplification of template molecule(s) using homogenous assay chemistries and workflows similar to those widely used for real-time PCR applications (Hinson et al., 2011, Anal. Chem. 83:8604-8610; Pinheiro et al., 2012, Anal. Chem. 84: 1003-1011). Once droplets are generated, they can be transferred on a PCR plate and emulsified PCR reactions can be run on a thermal cycler under a classical program such as for example described in the Biorad's Guideline for ddPCR (http://www.bio-rad.com/webroot/web/pdf/lsr/literature/Bulletin_6407.pdf).

Examples of droplet digital PCR systems include the QX100™ Droplet Digital PCR System by Bio-Rad, which partitions samples containing nucleic acid template into 20,000 nanoliter-sized droplets; and the RainDrop™ digital PCR system by RainDance, which partitions samples containing nucleic acid template into 1,000,000 to 10,000,000 picoliter-sized droplets.

**Method for identifying a cancer patient presenting microsatellite unstable tumors.**

The detection of a mutation in at least one, preferably two, three or four microsatellite sequences selected from the group consisting of: M1, M2, M3 and M4 is predictive factor for MSI phenotype and is indicative that a cancer patient presents microsatellite unstable sequence and is likely to benefit from immunotherapy (e.g., immune checkpoint inhibitors). On the contrary, if no mutation is detected in said microsatellite sequences, then said cancer patient has a MSS phenotype and is not likely to benefit from immunotherapy.

Therefore, the detection of at least one mutated microsatellite sequence according to the method as mentioned above can be used in the diagnosis of cancers, in particular of cancers which are associated with impaired DNA mismatch repair and notably of MSI positive cancers (or tumors).

In a particular embodiment, the present disclosure relates to a method for identifying a cancer patient presenting microsatellite unstable tumors who is likely to benefit from immunotherapy comprising the step of detecting a mutation in a cancer patient sample within at least one, preferably two, three or four microsatellite sequence selected from the group consisting of: M1, M2, M3 and M4 and determining a copy number or MAF of each microsatellite sequence(s) in said sample as described above.

As used herein, the term "microsatellite unstable tumors" has its general meaning in the art and refers to cancer liable to have a microsatellite instability (MSI) phenotype. "A cancer liable to have a MSI phenotype" refers to a sporadic or hereditary cancer in which microsatellite instability may be present (MSI, Microsatellite Instability) or absent (MSS, Microsatellite Stability).

"Microsatellite Instability" (hereinafter, "MSI"), as used herein, refers to a form of genetic instability in which alleles of genomic DNA obtained from certain tissue, cells, or bodily fluids of a given subject are mutated at a microsatellite sequence.

Examples of cancers liable to have a MSI phenotype include adenoma or primary tumors, such as colorectal cancer (also called colon cancer or large bowel cancer), colon adenocarcinoma, rectal adenocarcinoma, gastric cancer, stomach cancer, endometrial cancer, uterine cancer, uterine corpus endometrial carcinoma, breast cancer, bladder cancer, hepatobiliary tract cancer, liver hepatocellular carcinoma, urinary tract cancer, urothelial carcinoma, ovary cancer, ovarian serous cystadenocarcinoma, lung adenocarcinoma, lung squamous cell carcinoma, bladder cancer, prostate cancer, kidney cancer, kidney renal papillary cell carcinoma, head and neck cancer, skin cancer, skin cutaneous melanoma, thyroid carcinoma, squamous cell carcinoma, lymphomas, leukemia, brain cancer, brain lower grade glioma, glioblastoma, glioblastoma multiforme, astrocytoma, neuroblastoma and cancers described in Ronald J Hause et al., Nat. Med 2016 (39).

A sporadic cancer liable to have a MSI phenotype may refer to a cancer due to somatic genetic alteration of one of the Mismatch Repair (MMR) genes MLH1, MSH2, MSH6 and PMS2. For example, a sporadic cancer liable to have a MSI phenotype can be a cancer due to de novo bi-allelic methylation of the promoter of MLH1 gene. A hereditary cancer liable to have a MSI phenotype may refer to a cancer that occurs in the context of Lynch syndrome or Constitutional Mismatch-Repair Deficiency (CMMR-D). A patient suffering from Lynch syndrome is defined as a patient with an autosomal mutation in one of the 4 genes MLH1, MSH2, MSH6, and PMS2. A patient suffering from CMMR-D is defined as a patient with a germline biallelic mutation in one of the 4 genes MLH1, MSH2, MSH6, and PMS2. The MSI phenotype is present across different cancer types such as described in Ronald J Hause et al., Nat. Med 2016 (39).

In some embodiments of the disclosure, the patient is suffering from a cancer, is in remission of a cancer, is under cancer treatment, or is at risk of suffering from a cancer notably based on family history. In some embodiments for example the patient has familial tumor predisposition.

Preferably according to the present disclosure, the cancer is a breast cancer, including primary and advanced breast cancer, notably metastatic breast cancer. Typically, the cancer can be an advanced breast cancer, notably an ER-positive breast cancer developed after the development of acquired secondary resistance to aromatase inhibitors.

The term "biological sample" or "sample" is generally obtained from a subject or from a population of subjects. A sample may be any biological tissue or fluid with which the detection of microsatellite instability of the present disclosure may be assayed. Frequently, a sample will be a "tumor sample" (i.e., a sample obtained or derived from a cancer patient to be tested). The sample may also be an archival sample with a known diagnosis, treatment, and/or outcome history. Examples of such samples include fluids, tissues, cell samples, organs, biopsies, etc. Preferred biological samples are tumor sample. Derived materials include, but are not limited to, cells (or their progeny) isolated from the sample, as well as nucleic acid molecules (DNA and/or RNA) extracted from the sample.

In a preferred embodiment, the sample is tumor DNA sample. The DNA from the DNA sample can be genomic DNA or DNA issued from reverse RNA transcription. The genomic DNA may be constitutional DNA, DNA originating from a tumor (i.e., tumor genomic DNA), notably a malignant tumor. In some embodiments, notably when the sample is a biological fluid, the DNA sample may contain cell-free circulating DNA (cfcDNA) or circulating DNA. Early studies have shown that tumor DNA is released into the circulation and is present in particularly high concentrations in plasma and serum in a number of different types of cancers (Leon et al., 1977 Cancer Res 37:646-650; Stroun et al., 1989 Oncology 46:318-322). Thus, DNA sample according to the invention can contain cell-free circulating tumor DNA (cfcDNA) or circulating tumor DNA (ctDNA). In another embodiment, the DNA sample contains cell-free fetal DNA. Due to its high sensitivity, the method of the disclosure is particularly well-suited to be used on plasma sample containing low concentration of circulating, or cell-free target DNA such as cell-free or circulating tumor DNA or fetal DNA. In some embodiments of the present invention, the DNA can be obtained from reverse transcription of an RNA sample.

Typically, a DNA sample according to the invention is obtained from a patient. The terms "subject" and "patient" are used interchangeably herein and refer to both human and non-human animals. As used herein, the term "patient" or "subject" denotes a mammal, such as a rodent, a feline, a canine, and a primate. Preferably, a patient or a subject according to the invention is a human.

According to the present disclosure, a mutation within at least one, preferably two, three or four microsatellite sequence(s) as described above in a sample (e.g., tumor DNA sample) can be detected by any methods known in the art such as non-limiting examples, DNA sequencing, PCR based-method such as RT-PCR, qPCR or digital PCR as described above, preferably droplet digital PCR.

In a preferred embodiment, the present disclosure relates to a method for identifying a cancer patient presenting microsatellite unstable tumors who is likely to benefit from immunotherapy said method comprises the step of detecting a mutation within at least one, preferably at least two, three or four microsatellite sequence(s) selected from the group consisting of: M1, M2, M3 and M4 as described above in a plurality of nucleic acid molecules of a tumor DNA sample by subjecting said sample to a digital polymerase chain reaction as described above. In a preferred embodiment, said digital PCR is a droplet digital PCR. Preferably, a mutation is detecting in at least two mutated microsatellite sequences selected from the group consisting of: M1, M2, M3 and M4 as described above. In a particular embodiment, said digital PCR is performed by submitting the DNA sample to a pair of primers, MS probes and REF probes as described in Table 1.

According to the present disclosure, the method for identifying a cancer patient presenting microsatellite unstable tumors who is likely to benefit from immunotherapy further comprises a step of determining a copy number of each mutated microsatellite sequence(s) in said tumor DNA sample as described above.

In a preferred embodiment, the copy number of a mutated microsatellite sequence selected from the group consisting of M1, M2, M3 and M4 is determined for each microsatellite sequences and each value is compared to a corresponding threshold value.

A higher copy number of at least one, preferably two, three or four mutated microsatellite sequence(s) in comparison to corresponding threshold value(s) is indicative that the cancer patient presents microsatellite unstable tumor and is likely to benefit from immunotherapy. In a preferred embodiment, a higher copy number of at least two mutated microsatellite sequences in comparison to the corresponding threshold values is indicative that the cancer patient presents microsatellite unstable tumor and is likely to benefit from immunotherapy.

Preferably, the method comprises the steps of i) determining a copy number of each corresponding wild-type microsatellite sequence(s) in said tumor DNA sample and, ii) calculating a mutant allelic frequency (MAF) for each microsatellite sequence(s) with the following formula: MAF = copy number of mutated microsatellite sequence / (copy number of wild-type microsatellite sequence + copy number of mutated microsatellite sequence).

According to the present disclosure, a higher MAF value of at least one, preferably two, three or four microsatellite sequence(s) as described above as compared to corresponding threshold value(s) is indicative that the patient is likely to benefit from immunotherapy. In a preferred embodiment, a higher MAF value of at least two microsatellite sequences as described above as compared to corresponding threshold values is indicative that the patient is likely to benefit from the immunotherapy.

According to the present disclosure, the term "threshold value", "control value" or "cut-off value" can be determined experimentally, empirically, or theoretically. A threshold value refers to the copy number or MAF value of each mutated microsatellite sequence in a biological sample obtained from a general population or from a selected population of subjects. For example, the general population may comprise apparently healthy subjects, such as individuals who have not previously had any sign or symptoms indicating the presence of cancer. The term "healthy subjects" as used herein refers to a population of subjects who do not suffer from any known condition, and in particular, who are not affected with any cancer. In another embodiment, the threshold value refers to the copy number or MAF value of each mutated microsatellite sequence in a biological sample obtained from cancer patients who is not diagnosed with a MSI positive tumor or with a disease associated with a mutation in the DNA mismatch repair. Typically, the optimal sensitivity and specificity (and so the threshold value) can be determined using a Receiver Operating Characteristic (ROC) curve based on experimental data. For example, after determining the copy number of mutated microsatellite sequence or MAF value in a group of reference, one can use algorithmic analysis for the statistic treatment of the measured values in samples to be tested, and thus obtain a classification standard having significance for sample classification. In a particular embodiment, Receiver operating characteristic (ROC) analysis was performed to calculate the copy number of each mutated microsatellite sequence or MAF cut-off value of each microsatellite using tumor DNA samples with known microsatellite status. The copy number or MAF values offering the highest sensitivity and specificity were selected as cut-off points as described in the examples of the present application. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc. According to the present disclosure, the threshold value can be determined for each microsatellite sequences selected from the group consisting of: M1, M2, M3 and M4.

**Therapeutic use of an immunotherapy**

In another particular embodiment, the present disclosure relates to the therapeutic use of an immunotherapy (e.g. immune checkpoint inhibitor) in a cancer patient in need thereof wherein said immunotherapy (e.g. immune checkpoint inhibitor) is administered to said patient who is previously identified as a cancer patient presenting microsatellite unstable tumors, who is likely to benefit from immunotherapy in a method as described above.

Immunotherapy is a type of cancer treatment that activates the immune system to fight disease such as cancer. Immunotherapy that can be used to treat cancer includes as non-limiting examples: immune checkpoint inhibitors which are drugs that block immune checkpoint protein, T-cell transfer therapy, therapeutic vaccines, monoclonal antibodies or immune system modulators.

As used herein the term "immune checkpoint protein" has its general meaning in the art and refers to a molecule that is expressed by T cells and NK cells in that either turn up a signal (stimulatory checkpoint molecules) or turn down a signal (inhibitory checkpoint molecules). Immune checkpoint molecules are recognized in the art to constitute immune checkpoint pathways similar to the CTLA-4 and PD-1 dependent pathways (see e.g. Pardoll, 2012. Nature Rev Cancer 12:252-264; Mellman et al., 2011. Nature 480:480- 489).

As used herein, the term "immune checkpoint inhibitor" has its general meaning in the art and refers to any compound inhibiting the function of an immune inhibitory checkpoint protein. Inhibition includes reduction of function and full blockade. In particular, the immune checkpoint inhibitor particularly suitable for enhancing the proliferation, migration, persistence and/or cytotoxic activity of CD8+ T cells in the patient and in particular the tumor-infiltrating of CD8+ T cells of the patient.

In an embodiment, said immune checkpoint inhibitor of adaptive immune cells (T and B lymphocytes) is selected from the group consisting of anti-PDL1, anti-PD1, anti-SIRPA, anti-CTLA4, anti-CD137, anti-CD2, anti-CD28, anti-CD40, anti-HVEM, anti-BTLA, anti-CD160, anti-TIGIT, anti-TIM-1/3, anti-LAG-3, anti-2B4, and anti-OX40, anti-CD40 agonist, CD40-L, TLR agonists, anti-ICOS, ICOS-L and B-cell receptor agonists, in particular selected from the group consisting of anti-PDL1, anti-PD1 and anti-CD137. In a particular embodiment of the disclosure, the immune checkpoint inhibitor is an anti-PDL1 antibody.

In a particular embodiment, examples of immune checkpoint inhibitors are inhibitors that affect the PD/PDL-1 and CTLA-4 pathways and can be selected from the group consisting of: Ipilimumab, Nivolumab, Pembrolizumab, Atezolizumab, Avelumab and durvalumab.

In a particular embodiment, the present disclosure relates to a method of treating a cancer in a patient in need thereof, comprising:

i) detecting a mutation within at least one, preferably two, three or four microsatellite sequence(s), in a plurality of nucleic acid molecules of a DNA sample previously collected from said patient, preferably by subjecting said sample to a digital polymerase chain reaction, more preferably droplet digital PCR, wherein said microsatellite sequence is selected from the group consisting of: M1 M2, M3 and M4,

ii) determining a copy number or MAF value of each microsatellite sequence(s) wherein a higher copy number or MAF value of at least one mutated microsatellite sequence, preferably at least two, three or four mutated microsatellite sequences as compared to corresponding threshold value(s) is indicative that the patient is likely to benefit from immunotherapy, and

iii) administering a therapeutically efficient amount of an immunotherapy (e.g. immune checkpoint inhibitor) in said patient identified as likely to benefit from an immunotherapy.

The present disclosure relates to the use of immune checkpoint inhibitor in the manufacture of a medicament for the treatment of cancer in a patient who is previously identified as likely to benefit from immunotherapy in a method as described above.

Examples of cancers according to the present disclosure include as non-limiting examples adenoma or primary tumors, such as colorectal cancer (also called colon cancer or large bowel cancer), colon adenocarcinoma, rectal adenocarcinoma, gastric cancer, stomach cancer, endometrial cancer, uterine cancer, uterine corpus endometrial carcinoma, breast cancer, bladder cancer, hepatobiliary tract cancer, liver hepatocellular carcinoma, urinary tract cancer, urothelial carcinoma, ovary cancer, ovarian serous cystadenocarcinoma, lung adenocarcinoma, lung squamous cell carcinoma, bladder cancer, prostate cancer, kidney cancer, kidney renal papillary cell carcinoma, head and neck cancer, skin cancer, skin cutaneous melanoma, thyroid carcinoma, squamous cell carcinoma, lymphomas, leukemia, brain cancer, brain lower grade glioma, glioblastoma, glioblastoma multiforme, astrocytoma, neuroblastoma and cancers described in Ronald J Hause et al., Nat. Med 2016 (39).

In some embodiments of the disclosure, the patient is suffering from a cancer, is in remission of a cancer, is under cancer treatment, or is at risk of suffering from a cancer notably based on family history. In some embodiments for example the patient has familial tumor predisposition.

Preferably according to the present disclosure, the cancer is a breast cancer, including primary and advanced breast cancer, notably metastatic breast cancer. Typically, the cancer can be an advanced breast cancer, notably an ER-positive breast cancer developed after the development of acquired secondary resistance to aromatase inhibitors.

In the context of the present disclosure, the term "treating" or "treatment", as used herein, means reversing, alleviating, inhibiting the progress of, or preventing the disorder or condition to which such term applies, or reversing, alleviating, inhibiting the progress of, or preventing one or more symptoms of the disorder or condition to which such term applies.

As used herein, a "therapeutically effective amount" or an "effective amount" means the amount of a composition that, when administered to a subject for treating a state, disorder or condition is sufficient to effect a treatment. The therapeutically effective amount will vary depending on the compound, formulation or composition, the disease and its severity and the age, weight, physical condition and responsiveness of the subject to be treated.

The immunotherapy (e.g. immune checkpoint inhibitor) described herein may be administered by any means known to those skilled in the art, including, without limitation, intravenously, orally, intra-tumoral, intra-lesional, intradermal, topical, intraperitoneal, intramuscular, parenteral, subcutaneous and topical administration. Thus, the compositions may be formulated as an injectable, topical, or ingestible formulation. Administration of the compounds or therapeutic agents to a subject in accordance with the present disclosure may exhibit beneficial effects in a dose-dependent manner. Thus, within broad limits, administration of larger quantities of the compositions is expected to achieve increased beneficial biological effects than administration of a smaller amount. Moreover, efficacy is also contemplated at dosages below the level at which toxicity is seen.

It will be appreciated that the specific dosage of an immunotherapy (e.g. immune checkpoint inhibitor) administered in any given case will be adjusted in accordance with the composition being administered, the volume of the composition that can be effectively delivered to the site of administration, the disease to be treated or inhibited, the condition of the subject, and other relevant medical factors that may modify the activity of the compositions or the response of the subject, as is well known by those skilled in the art.

For example, the specific dose of an immunotherapy (e.g. immune checkpoint inhibitor) for a particular subject depends on age, body weight, general state of health, diet, the timing and mode of administration, the rate of excretion, medicaments used in combination and the severity of the particular disorder to which the therapy is applied. Dosages for a given patient can be determined using conventional considerations, e.g., by customary comparison of the differential activities of the compositions described herein and of a known agent, such as by means of an appropriate conventional pharmacological protocol. The compositions can be given in a single dose schedule, or in a multiple dose schedule.

Suitable dosage ranges for an immunotherapy (e.g., immune checkpoint inhibitor) may be of the order of several hundred micrograms of the agent with a range from about 0.001 to 10 mg/kg, preferably with the range from about 0.01 to 1 mg/kg, more preferably from about 1 to 10 mg/kg, again more preferably 10 mg/kg.

**Method for evaluating therapeutic response of an immunotherapy**

The detection of a mutation in at least one, preferably two, three or four microsatellite sequence(s) according to the present disclosure makes it possible to classify the patient as a responder of an immunotherapy, in particular responder to an immune checkpoint inhibitor treatment by determining a copy number or MAF of each mutated microsatellite sequence(s) in a sample (e.g. cell-free circulating tumor DNA sample) and by determining whether the copy number or MAF of at least one, preferably two, three, four microsatellite sequence(s) is increased or decreased during the treatment, preferably in comparison to a copy number or MAF value of corresponding microsatellite sequence in a DNA sample obtained from said cancer patient at a prior time point of the treatment, preferably prior to the administration of at least one immunotherapy

The present disclosure relates to an *in vitro* method for evaluating the therapeutic response to an immunotherapy in a patient having a cancer, said method comprising:

   i) detecting a mutation within at least one, preferably at least two, three or four microsatellite sequence(s) in a plurality of nucleic acid molecules of a tumor DNA sample of a patient having received at least one dose of an immunotherapy (e.g. immune checkpoint inhibitor) by subjecting said sample to a digital polymerase chain reaction, wherein said microsatellite sequence is selected from the group consisting of: M1, M2, M3 and M4,
   ii) determining a copy number or MAF of each microsatellite mutated sequence(s) in said tumor DNA sample,

wherein a decrease of a copy number or MAF of at least one mutated microsatellite sequence in the patient sample during the treatment is indicative that the patient is responsive to the immune checkpoint treatment.

The term "responder, or responsive to a treatment" refers to a subject in whom the onset of at least one of the symptoms of the condition to be treated is delayed or prevented, upon or after treatment, or whose symptoms or at least one of the symptoms stabilize, diminish or disappear.

"Therapeutic response" refers to the consequence of a medical treatment in a patient, the results of which are judged to be useful or favorable. For instance, a therapeutic response may be the delay or the prevention of at least one of the symptoms, upon or after treatment, or may be that symptoms or at least one of the symptoms in patient stabilize, diminish or disappear.

According to the present disclosure, by therapeutic response in patient treated with immunotherapy such as immune checkpoint inhibitor, it is meant that the performed steps of immunotherapy such as immune checkpoint inhibitor administration result in improving the clinical condition of an animal or a human patient in need thereof, who suffers from cancers. Such treatment aims at improving the clinical status of the animal or human patient, by eliminating or lowering the symptoms associated with cancers.

The term "evaluating therapeutic response to treatment", as used herein, refers to an ability to assess whether the treatment of a patient is likely effective in (e.g., providing a measurable benefit or positive medical response to) the patient after some time of administration of the treatment. In other terms, according to the present disclosure, evaluating the response to immunotherapy refers to an ability to assess whether a cancer patient is likely responsive to immunotherapy.

According to the present method for evaluating therapeutic response, the sample is previously collected from a patient having received a dose of immunotherapy, in particular immune checkpoint inhibitor. According to the present disclosure, a patient having received immunotherapy refers to a patient having received at least one dose of immunotherapy. The therapeutic response can be evaluated according to the present method, after each administration of dose of immunotherapy (e.g., immune checkpoint inhibitor) throughout the course of treatment for monitoring the therapeutic response over time.

In a particular embodiment, the copy number or MAF value of each microsatellite sequence in a sample of a patient having received immunotherapy is compared to a threshold value. In a preferred embodiment, the threshold value refers to the copy number or MAF value of each microsatellite sequence in a biological sample obtained from said cancer patient monitored at a different time, preferably at a prior time point of the treatment.

A decrease of a copy number of at least one, preferably two, three or four mutated microsatellite sequence(s) selected from the group consisting of M1, M2, M3 and M4 in a sample of cancer patient having received at least one first dose of immunotherapy (e.g. immune checkpoint inhibitor) during the treatment, preferably in comparison to a copy number value or MAF value of corresponding microsatellite sequence(s) in a DNA sample obtained from said cancer patient at a prior time point of the treatment, preferably prior to the administration of at least one immunotherapy, correlates with therapeutic response of said cancer patient to immunotherapy.

In a preferred embodiment, a decrease of MAF of at least one, preferably two, three or four mutated microsatellite sequence(s) selected from the group consisting of M1, M2, M3 and M4 in a sample of cancer patient having received at least one first dose of immunotherapy (e.g. immune checkpoint inhibitor) during the treatment, preferably in comparison to a copy number value or MAF value of corresponding microsatellite sequence(s) in a DNA sample obtained from said cancer patient at a prior time point of the treatment, preferably prior to the administration of at least one immunotherapy, correlates with therapeutic response of said cancer patient to immunotherapy.

If after immunotherapy, the copy number of at least one, preferably two, three or four mutated microsatellite sequences selected from the group consisting of M1, M2, M3 and M4 in sample of cancer patient is increased during the treatment, the treatment should be interrupted or modified. The method for evaluating therapeutic response according to the present disclosure can indicate success or failure of immunotherapy to a cancer patient.

In another aspect, the MSI phenotype of the cancer (i.e., positive or negative) has important implications in cancer prognosis and rational planning of treatment (Boland and Goel, Gastroenterology 2010). Therefore, even in the case of cancers with low MSI positive prevalence, it remains of high relevance to identify whether the patient is suffering from a MSI positive tumor or a MSI negative tumor. The method of the present invention can therefore be used in the prognosis of various cancers. Identification of a positive MSI cancer is generally associated with a better prognosis.

Thus, the present disclosure also relates to a method for the prognosis of cancers comprising the detection of a mutation within at least one microsatellite sequence selected from the group consisting of: M1, M2, M3 and M4 in a DNA sample according to the present disclosure.

Kit

The present disclosure also encompasses a kit for identifying a mutation, preferably for determining the copy number of a mutation in at least one microsatellite sequence selected from the group consisting of M1, M2, M3 and M4 in a DNA sample.

According to the present disclosure, said kit comprises:

- a pair of primers for amplifying a target sequence including a mutated or wild-type microsatellite sequence selected from the group consisting of M1, M2, M3 and M4,
- an oligonucleotide probe (MS) comprising a sequence complementary to the wild-type microsatellite sequence and,
- an oligonucleotide reference probe (REF) comprising a sequence complementary to a part of said target sequence located outside of said microsatellite sequence.

In a preferred embodiment said kit comprises:

- a pair of primers of SEQ ID NO: 1 and 2, the oligonucleotide microsatellite probe (MS) of SEQ ID NO: 3 and oligonucleotide reference probe (REF) of SEQ ID NO: 4,
- a pair of primers of SEQ ID NO: 5 and 6, the oligonucleotide microsatellite probe (MS) of SEQ ID NO: 7 and oligonucleotide reference probe (REF) of SEQ ID NO: 8,
- a pair of primers of SEQ ID NO: 9 and 10, the oligonucleotide microsatellite probe (MS) of SEQ ID NO: 11 and oligonucleotide reference probe (REF) of SEQ ID NO: 12 and/or
- a pair of primers of SEQ ID NO: 13 and 14, the oligonucleotide microsatellite probe (MS) of SEQ ID NO: 15 and oligonucleotide reference probe (REF) of SEQ ID NO: 16.

In a preferred embodiment, said MS and REF probes are labelled with different fluorescent molecules.

In some embodiments, said kit can further comprises a thermostable DNA polymerase. Thermostable DNA polymerases are typically described in Newton and Graham 1994 In: PCR, BIOS Scientific Publishers, Ltd., Oxford, UK. 13. Advantageously, the thermostable polymerase is the Taq polymerase.

Primers, probes, target fragment and microsatellite sequences usable in a kit according to the disclosure have been previously described.

The kit as above mentioned can be used in the clinical applications as previously described.

The invention will now be exemplified with the following examples, which are not limitative, with reference to the attached drawings in which:

## FIGURE LEGENDS

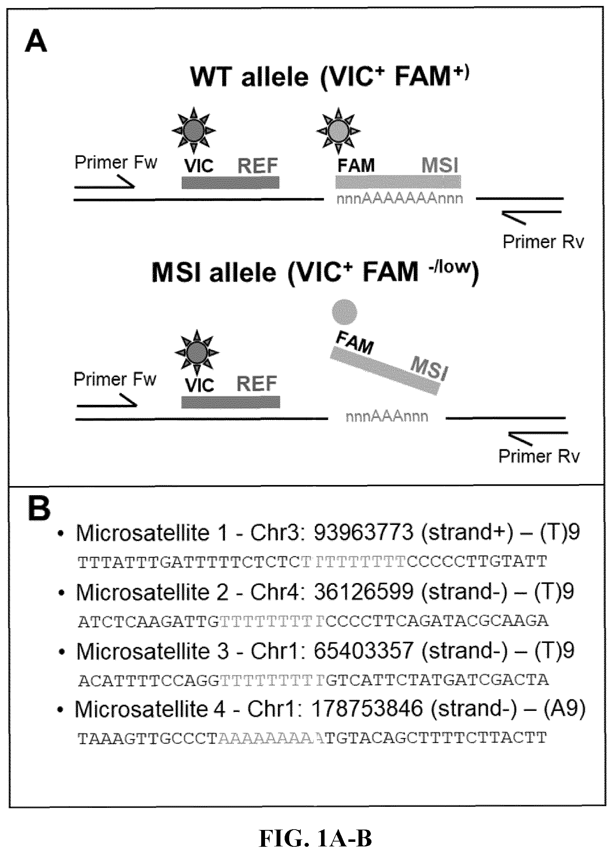

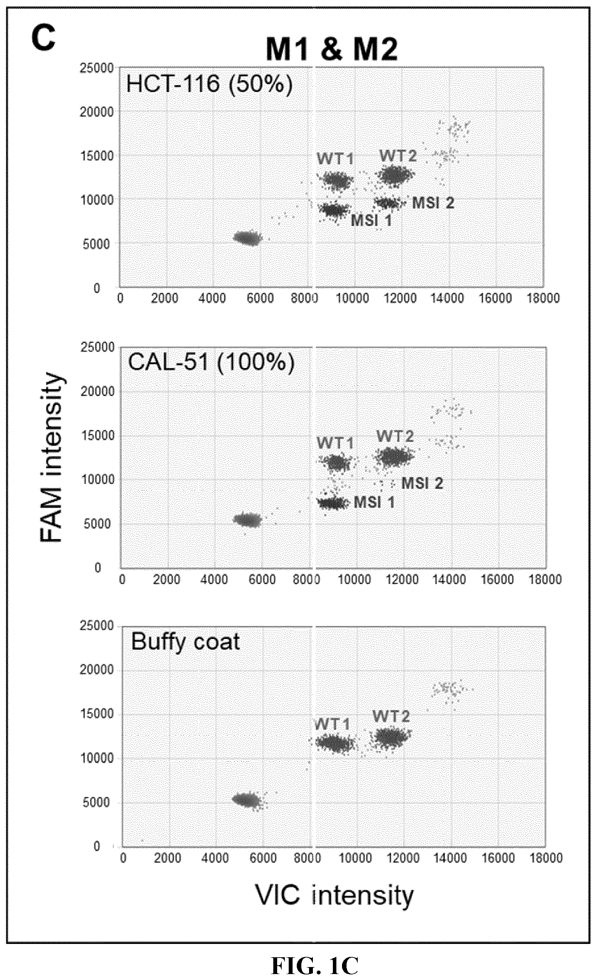

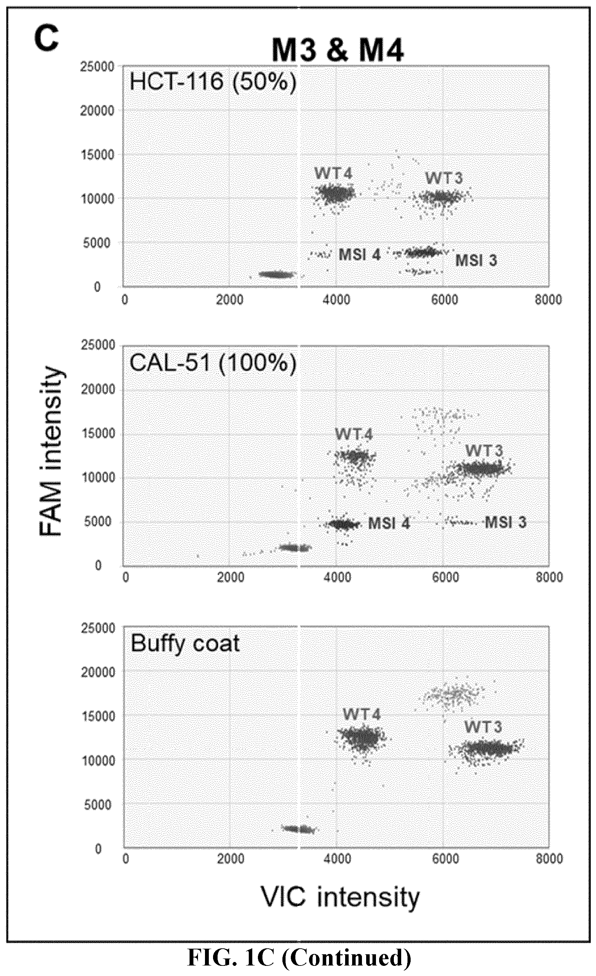

**Figure 1.** Microsatellite instability detection by drop-off ddPCR assays. A) the principle of drop-off ddPCR assays (adapted from (Silveira AB, et al. Clin Chem. 2020;66:606-13). B) DNA sequences (SEQ ID: NO 17, 18, 19 and 20) and genomic coordinates (GRCh37) of the 4 selected microsatellites. C) 2D scatter plots of multiplex M1&M2 and M3&M4 drop-off ddPCR assays. DNA from HCT-116 (diluted two times in WT genomic DNA) and CAL-51 cell lines were used as MSI-H positive controls and WT genomic DNA were used as MSS control. Droplets containing wild type (WT), MSI alleles (MSI) and no DNA template are indicated. Droplets co-capturing different target molecules are indicated as light grey clusters. The mutant allelic frequencies (MAFs) of each microsatellite are: M1: 49.80%, M2: 22.60%, M3: 47.00%, M4: 2.82% for HCT-116 and M1: 52.50%, M2: 0.98%, M3: 2.94%, M4: 54.70% for CAL-51.

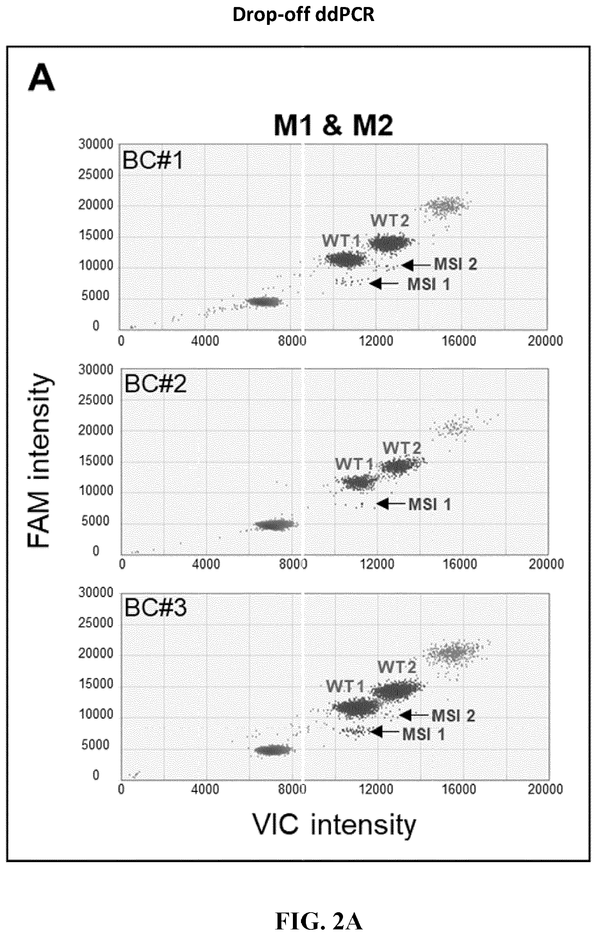

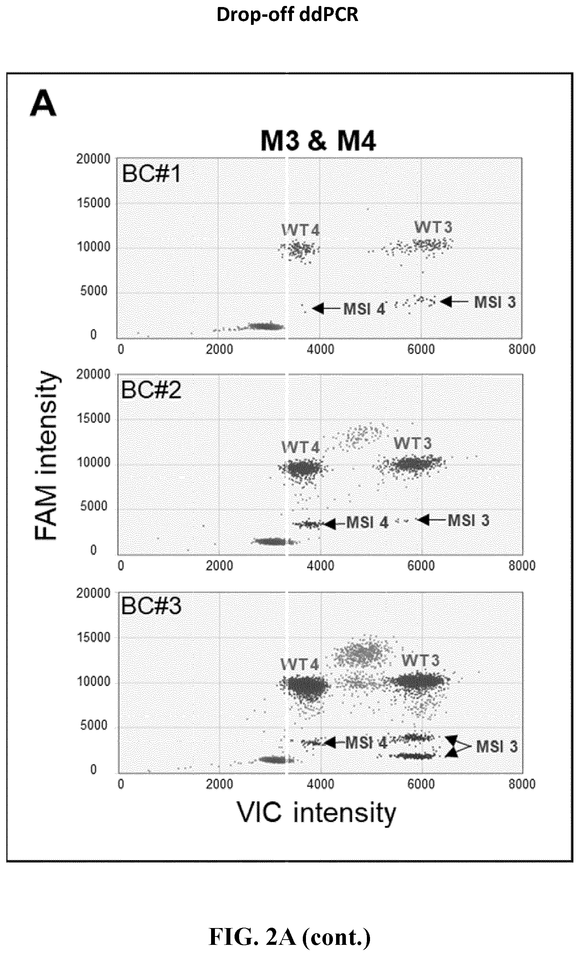

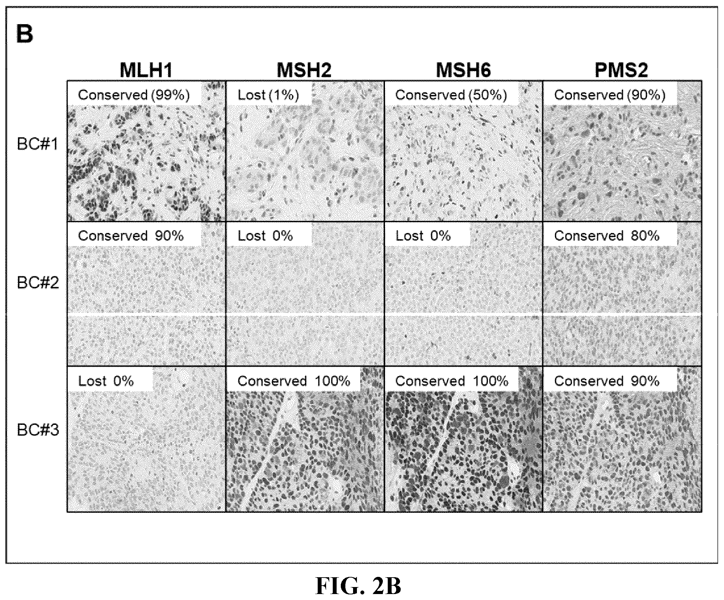

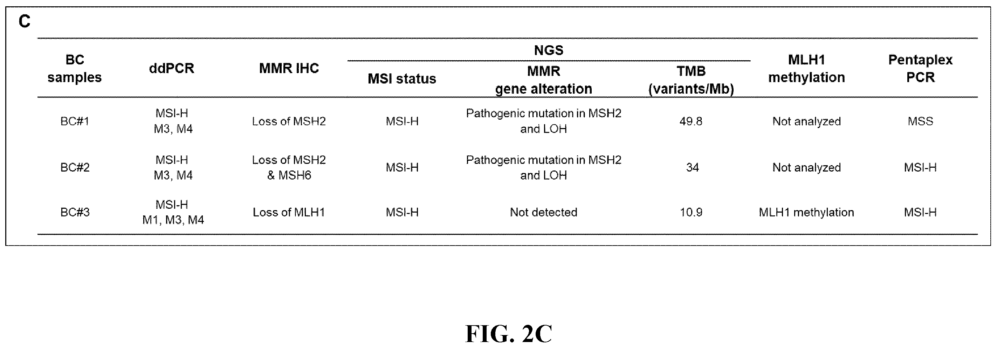

**Figure 2.** MSI detection in 3 breast tumors previously known as MSI-H based on NGS. A) 2D scatter plots of multiplex M1&M2 and M3&M4 drop-off ddPCR assays. The mutant allelic frequencies (MAFs) of each microsatellite are: M1: 0.94%, M2: 0.38%, M3: 16.20%, M4: 1.40% for BC#1; M1: 1.29%, M2: 0.00%, M3: 1.04%, M4: 8.88% for BC#2; M1: 1.86%, M2: 0.44%, M3: 25.3%, M4: 1.46% for BC#3. B) IHC staining showing the percentage of MMR protein expression in tumor cells. C) MSI status characterization by different MSI detection methods. MSI-H: microsatellite instability-high; MSS: microsatellite stable. TMB: Tumor Mutational Burden. LOH: loss of heterozygosity.

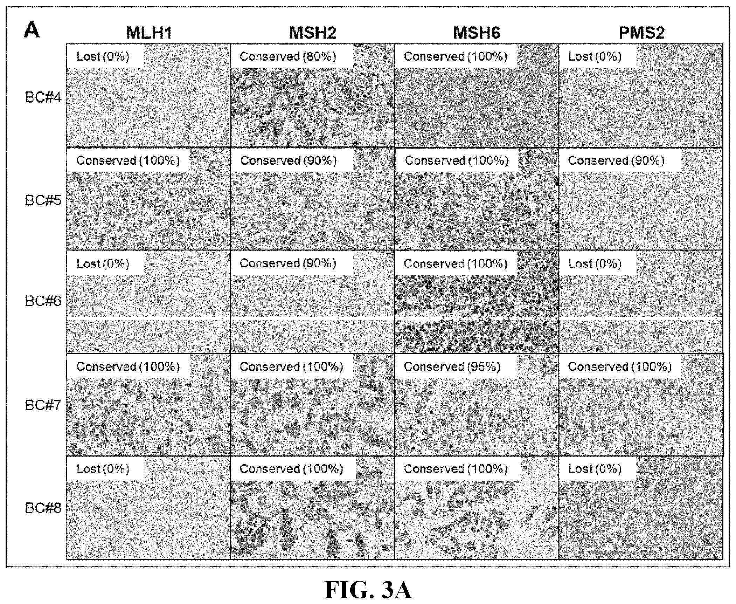

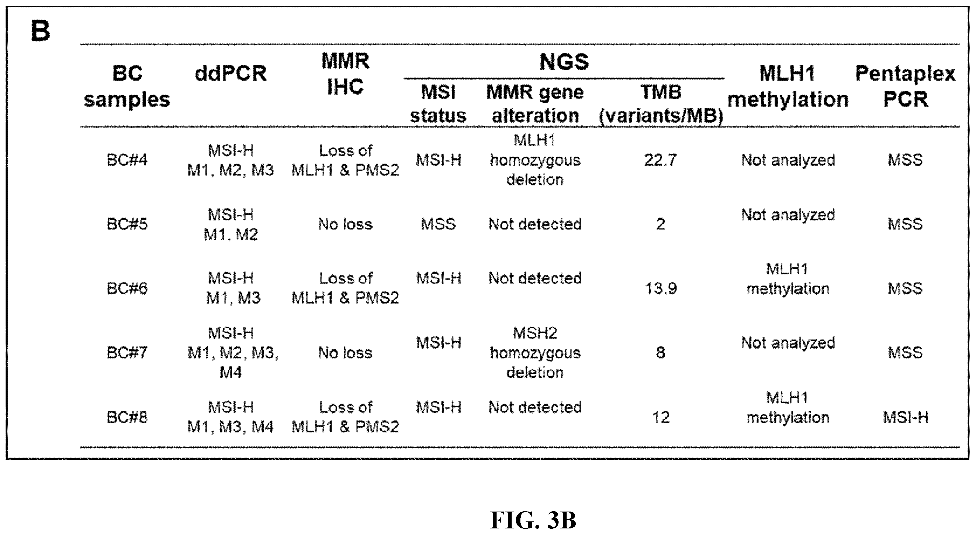

**Figure 3.** Characterization of MSI status by IHC, pentaplex PCR and NGS in the 5 breast tumors retrieved as MSI-H by drop-off ddPCR assays in the blind analysis. A) IHC staining showing the percentage of MMR protein expression in tumor cells. B) Comparison of MSI detection by different methods and the possible molecular mechanisms responsible for MSI-H in MMR genes. MSI-H: microsatellite instability-high; MSS: microsatellite stable. TMB: Tumor Mutational Burden.

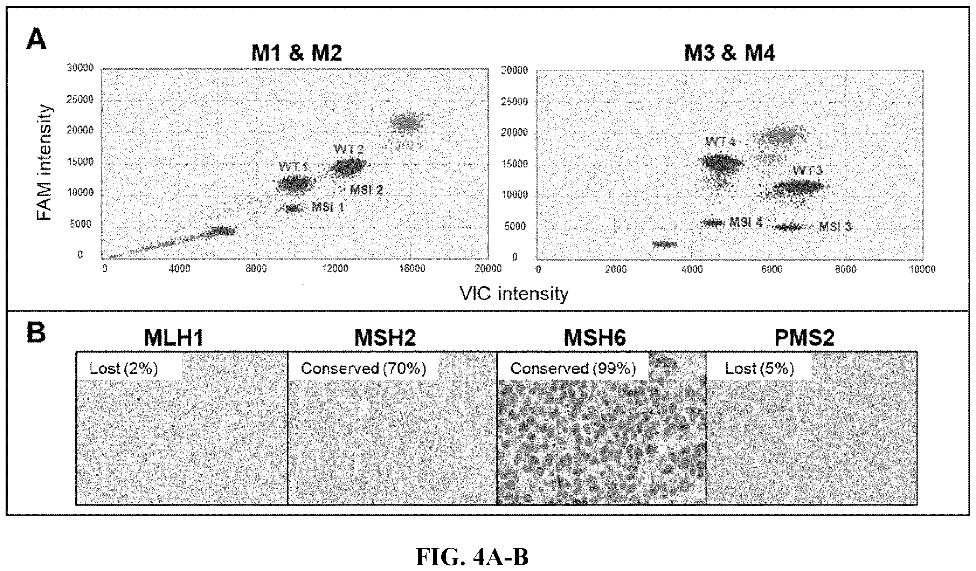

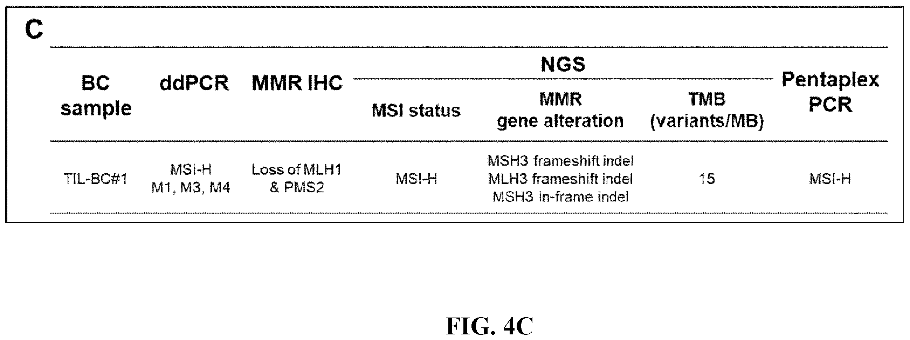

**Figure 4.** MSI detection in TILs-rich breast tumor (TIL-BC#1) retrieved as MSI-H by drop-off ddPCR assays. A) 2D scatter plots of multiplex M1&M2 and M3&M4 drop-off ddPCR assays. B) IHC staining showing the percentage of MMR protein expression in tumor cells. C) Comparison of MSI detection by different methods and the possible molecular mechanisms responsible for MSI-H in MMR genes. MSI-H: microsatellite instability-high. TMB: Tumor Mutational Burden.

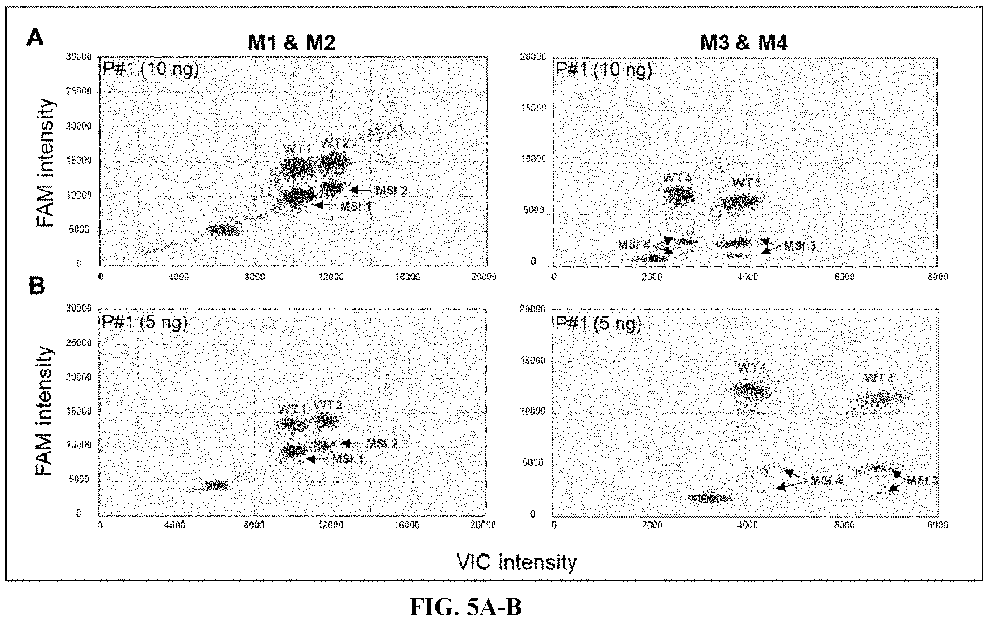

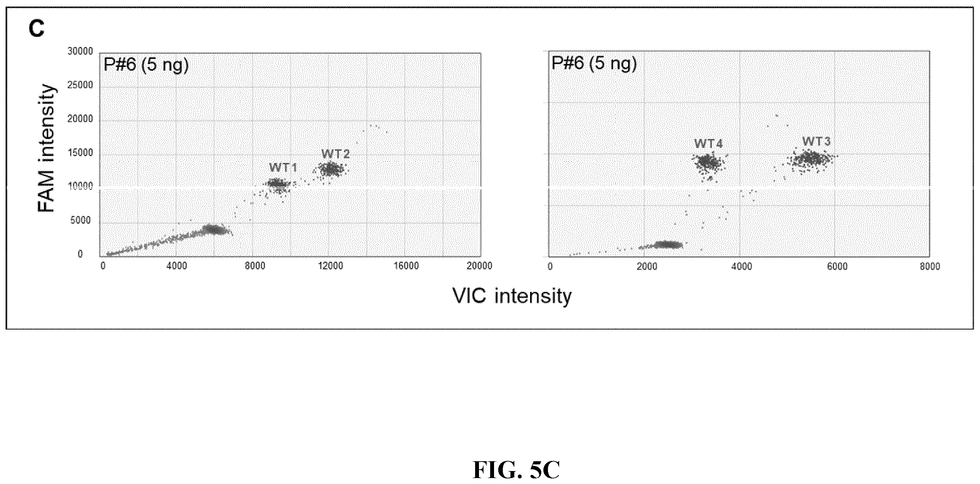

**Figure 5.** 2D scatter plots of MSI detection in cfcDNA by multiplex M1&M2 and M3&M4 drop-off ddPCR assays. (A-B) Metastatic BC cfcDNA (P#1) retrieved as MSI-H using 10 ng and 5 input respectively. C) Example of BC cfcDNA with no MSI-H detected (P#6). The mutant allelic frequencies (MAFs) of each microsatellite are: A) M1: 45.60%, M2: 27.60%, M3: 25.60%, and M4: 9.24%. B) M1: 49.69%, M2: 28.21%, M3: 27.44%, and M4: 8.23%. C) M1: 0.00%, M2: 0.00%, M3: 0.00%, and M4: 0.00%.

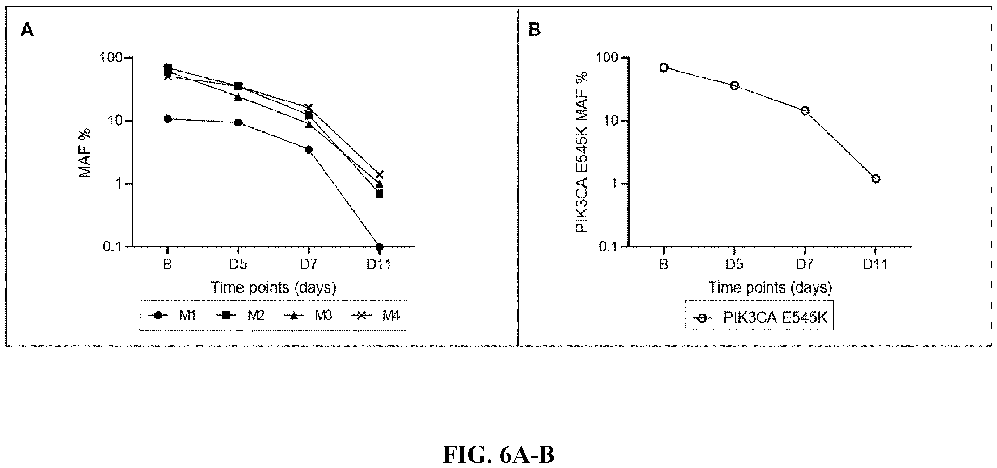

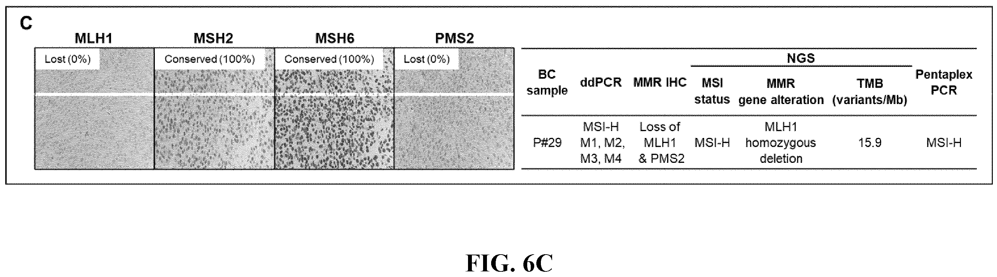

**Figure 6.** MSI detection in metastatic BC cfcDNA (P#29). Mutant allelic frequencies (MAPs) in cfcDNA at baseline (B) and during treatment with pembrolizumab, estimated by: A) MSI-ddPCR targeting 4 BC microsatellites, and B) ddPCR assay targeting PIK3CA E545K mutation. C) IHC staining showing the percentage of MMR protein expression in tumor cells and MSI status characterization by different methods MSI-H: microsatellite instability-high. TMB: Tumor Mutational Burden.

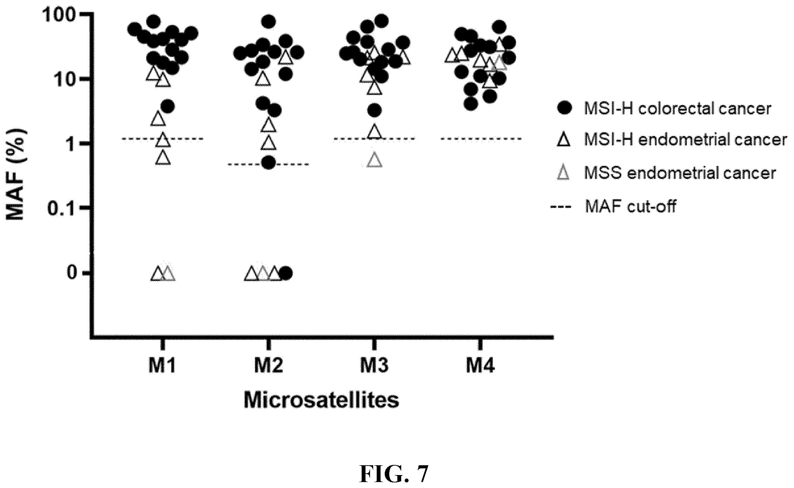

**Figure 7.** Microsatellite instability status characterization by M1, M2, M3 and M4 drop-off ddPCR assays in non-breast cancer tumors previously known as MSI-H (pentaplex test). Each dot or triangle represents the mutation allelic frequency (MAF) of the measured microsatellite. Black dots/triangles and grey triangles represent the samples classified as MSI-H or MSS by drop-off ddPCR assays, respectively. The MAF cut-off value for each microsatellite is represented by a discontinuous line.

## EXAMPLES

## Example 1

### 1. Material and methods

### Cell line and clinical samples

Human cancer cell lines: HCT-116 (colorectal cancer) and CAL-51 (breast cancer) were used as MSI-H controls. Buffy coats from healthy donors obtained from the Etablissement Français du Sang (EFS) following French and European ethical rules, were used as microsatellite stable (MSS) controls. Genomic DNA was extracted using the QIAamp DNA mini Kit (Qiagen) following the manufacturer's instructions. Archived clinical samples from BC patients with mixed subtypes (Formalin-Fixed Paraffin-Embedded (FFPE) or frozen tissue DNA, n=344; plasma cell-free circulating DNA, n=28) were retrieved from the Institut Curie Pathology and Genetics units. Frozen tumor tissue DNA from 11 triple negative BC patients with high density of tumor-infiltrating lymphocytes (TILs-rich) were obtained from the Institut Curie Biological Resource Center (CRB). In a prospective analysis, blood samples (n=4) were collected from a metastatic BC patient with a known MSI-H tumor (NGS data) before (baseline) and during pembrolizumab therapy.

All female patients included have been treated and followed at Institut Curie Hospitals between 2010 and 2022 and agreed for the use of archived tumor or plasma samples according to the law. A waiver of informed consent was obtained from the local Institutional Review Board due to the retrospective nature of the work.

### Plasma sample preparation, storage, DNA extraction and quantification

Blood samples from BC patients were collected in Cell-Free DNA BCT (Streck) tubes and plasma was isolated within 2 days using a two-step centrifugation: 1500g for 10 min then 16000g for 10 min at room temperature. DNA was extracted using the QIAmp Circulating Nucleic Acid Kit (Qiagen) according to the manufacturer's instructions and quantified by Qubit 2.0 fluorometer (Invitrogen, Life Technologies) using the dsDNA HS Assay (Invitrogen). Extracted DNA was stored at -20°C before use.

### Identification of new microsatellites for MSI detection in breast cancer tumors

Variant calling files (VCF) of 559 BC samples, including 10 dMMR tumors, were obtained from a publicly available whole-genome sequencing dataset (Davies H, et al. Cancer Res. 2017;77:4755-62). These dMMR tumors showed high numbers of insertions/deletions (indels) occurring at polynucleotide repeat tracts consistent with the pattern of microsatellite instability associated with MMR deficiency. The identification of MSI loci involved three major steps. First, microsatellite loci with deletions of less than 200 bp were selected (255824 loci) in the A/T context from the 10 dMMR/MSI-H tumors. Second, the microsatellites loci with deletions in at least 4 dMMR tumors and no alteration in the remaining 549 MMR proficient (pMMR)/MSS tumors were retained (104 microsatellite loci). Finally, using a combinatorial optimization analysis, the best microsatellite loci combination that could identify most dNMR tumors was selected (n=4 microsatellites). These microsatellites present a frequency of single nucleotide polymorphism (SNP) in the population less than 0.02%.

**MSI detection with the use of multiplex drop-off digital PCR assays**

Primers and probes are described in the Table 2.

**Table 2.** Primers and probes of the 4 identified microsatellites.

| MS | Genomic location (GRCh37) | Genomic region | MS type | Forward primer (5'-3') | Reverse primer (5'-3') | Drop-off probe (5'-3') | REF probe (5'-3') |
|---|---|---|---|---|---|---|---|
| M1 | Chr3:9396 3773 | intergenic | T(9) | AGTTCTCTG AATTTGCTCT GAATTA (SEQ ID NO : 1) | ACAAGTACA TCCATAGAG TTCAAC (SEQ ID NO : 2) | **FAM**-TCTC**TTTTTTTT** TCCC- **MGB**-**NFQ** (SEQ ID NO: 3) | **VIC**-CTGGAACTC TTATTATCT-**MGB-NFQ** (SEQ ID NO: 4) |
| M2 | Chr4:3612 6599 | intronic | T(9) | TGCATTTGTT TGTGACTGT TTTAC (SEQ ID NO : 5) | TCTTTCCTT GTCATCTTG CGT (SEQ ID NO : 6) | **FAM**-AGATTG**TTTTT TTTT**CC-**MGB-NFQ** (SEQ ID NO : 7) | **VIC**-CTGCTGATA GACAAATCC-**MGB-NFQ** (SEQ ID NO : 8) |
| M3 | Chr1:6540 3357 | intronic | T(9) | CTCGCTTTA AGGATATGT CTG (SEQ ID NO : 9) | GTGGAACAC TGATTAGTA ACTG (SEQ ID NO : 10) | **FAM**-CAGG**TTTTTTT TT**GTC-**MGB-NFQ** (SEQ ID NO : 11) | **VIC**-CTATGATCGA CTAAGT-**MGB-NFQ** (SEQ ID NO : 12) |
| M4 | Chr1:1787 53846 | intronic | A(9) | TGTGGAGCA AACAGAGGG AT (SEQ ID NO : 13) | TTGCTACTT ACCAAATTG AAGTGT (SEQ ID NO : 14) | **FAM**-CCCT**AAAAAA AAA**TGT-**MGB-NFQ** (SEQ ID NO : 15) | **VIC**-CTTACTTTTC CCACT-**MGB-NFQ** (SEQ ID NO : 16) |

Multiplex drop-off digital PCR assays (microsatellites M1 with M2, and microsatellites M3 with M4) were developed as previously reported (Figure 1A) (Silveira Ab et al. Clin Chem. 2020;66:606-13) and performed using the BioRad QX100 with 900 nM of each primer, 250 nM or 400nM of each probe, 0.5 units of Uracile-ADN glycosylase (UDG, Roche) under the following conditions: 40°C for 10 min (1 cycle); 95°C for 10 min (1 cycle); 94°C for 30 sec and 59 °C for 1 min (45 cycles), and 98°C for 1 min (1 cycle). Basically, the drop-off ddPCR design includes two hydrolysis probes within the same PCR fragment; the reference probe (REF) is complementary to a non-variable region, and the target probe (drop-off) is complementary to the WT sequence of the target microsatellite in which deletions are frequently observed. In the presence of a WT allele, REF and drop-off probes hybridize evenly, resulting in a double signal (VIC+/FAM+). Whereas in the presence of altered allele (insertion, deletion, mutation), the drop-off probe cannot hybridize resulting in a loss of FAM signal, while maintaining a VIC signal (VIC+/FAM-/ low).

Cluster thresholding and quantification were performed with QuantaSoft v.1.7.4. Droplets were manually assigned as wildtype (WT) or mutant (MSI) based on their fluorescence amplitude: WT: VIC+/FAM+; MSI mutant: VIC+/FAM- /low. The mutant allelic frequencies (MAPs) were calculated as follows: copy numbers of MSI mutant / (copy numbers of WT + copy numbers of MSI mutant).

### ddPCR data analysis

The false-positive rate of each assay was estimated using ≥40 replicates of WT DNA. The limit of blank (LOB) was defined as the upper 95% confidence limit of the mean false-positive measurements. The analytical sensitivity was estimated using serial dilutions of MSI-H cfcDNA in WT DNA, with MAFs ranging from 10 to 0.01 %. The number of replicates per dilution point ranged from 3 to 8. The limit of detection (LOD) was estimated as the lowest mutant ratio that could be reliably distinguished from the LOB value.

### MMR proteins immunohistochemistry and MSI detection using pentaplex PCR, MSI detection by NGS panel and MLH1 promotor methylation

IHC staining of MMR proteins (MLH1, MSH2, MSH6, PMS2) were performed on FFPE tissue sections by the department of Pathology of Institut Curie and read by senior pathologists. The following antibodies were used: MLH1 clone ES05 (Agilent Cat# IR079, RRID:AB_2877720), MSH2 clone FE11 (Agilent Cat# IR085, RRID:AB_2889974), MSH6 clone EP49 (Agilent Cat# IR086, RRID:AB_2889975), PMS2 clone A16-4 (BD Biosciences Cat# 556415, RRID:AB_396410). A tumor was called as dMMR if it presented a complete nuclear loss of expression of at least one MMR protein.

Pentaplex PCR targeting five microsatellites (BAT-25, BAT-26, NR-21, NR-24, and NR-27), followed by capillary electrophoresis was performed using MSI Analysis System (v1.2, Promega) by the pharmacogenomics service of Institut Curie. The tumor was classified as MSI-H if the instability was present in two or more microsatellites.

MSI status was analyzed using a large capture-based targeted NGS panel ("DRAGON") developed at Institut Curie for molecular analysis of tumors. This panel comprises 571 sequence regions including 4 MMR components (MLH1, MSH2, MSH6, PMS2) and 86 microsatellite loci. NGS libraries were prepared using Agilent SureSelect XT-HS kit. Following this, sequencing was performed on NovaSeq (Illumina) at the Institut Curie core genomic facility with a mean reading depth of 1500X and a minimal depth of 300X. Variant calling was performed using VARSCAN2 v2.4.3 (RRID:SCR_006849) and MSI status was analyzed using MSIsensor v2 (RRID:SCR_006418). As per routine standards, tumors are classified as MSI-H if the instability was present in at least 5 microsatellite loci. The DRAGON panel covers 2.7 megabase and allows estimating the tumor mutational burden (TMB), a measurement of the number of mutations per megabase in tumor cells.

MLH1 methylation level in its promoter region was investigated on MSI-H tumors presenting loss of MLH1 protein expression by IHC. Tumor DNA was first converted by bisulfite treatment using the EZ DNA Methylation Direct kit (ZYMO research) and then analyzed using the EpiMelt MLH1 test assay (MethylDetect).

### Statistical analysis

Receiver operating characteristic (ROC) analysis was performed to calculate the MAF cut-off value of each microsatellite using BC samples with known microsatellite status (detected by DRAGON NGS panel, MSS, n=133; MSI-H, n=8). The MAF values offering the highest sensitivity and specificity were selected as cut-off points: 1.17% for M1, 0.51% for M2, 1.11% for M3 and 1.26% for M4.

### 2. Results

2.1 Microsatellites identification and multiplex drop-off ddPCR assays optimization.

From the WGS dataset of 559 BC samples (549 pMMR/MSS, 10 dMMR/MSI-H), 104 microsatellites loci harboring deletions in at least 4 dMMR/MSI-H tumors and no indels in the remaining pMMR/MSS tumors were retrieved. A combinatorial optimization analysis was performed to select a panel of microsatellites with a limited number but offering the highest detection sensitivity and specificity in combination. Finally, 4 microsatellites (M1, M2, M3, M4) were identified, their combination displaying a sensitivity of 90% and a specificity of 100% towards MSI-H status determination in the BC samples of the WGS dataset. Genomic coordinates and DNA sequences of these 4 microsatellites are shown in **Figure 1B.** Two multiplex drop-off ddPCR assays for the 4 microsatellites (M1 with M2, and M3 with M4) were then developed. Human cell lines (HCT-116 and CAL-51) were used as MSI-H positive controls and genomic DNA from healthy donors was used as MSS control. As shown in **Figure 1C,** a clear separation between WT and MSI droplet clusters was observed for these 4 microsatellites in both cell lines. No MSI was detected in genomic DNA from healthy donors. Similarly, to the pentaplex assay, samples were considered as MSI-H in this study if two or more microsatellites presented MAF values higher than the defined cut-offs. This criterion was used to avoid misclassification of MSS samples as MSI-H due to germline polymorphisms.

2.2 Analytical performance of the developed drop-off ddPCR assays for MSI detection.

To estimate the analytical sensitivity of each MSI-ddPCR assay, the inventors subsequently used serial dilutions (from 10 to 0.01%) of a circulating tumor DNA (ctDNA) from a known MSI-H colorectal cancer tumor in genomic DNA from healthy donors. This ctDNA was also found MSI-H with the present assays (4 microsatellites unstable). The limit of blank (LOB) was estimated at 0.04% for both M1 and M2, 0.03% for M3, and 0.15% for M4. The limit of detection (LOD) was found to be 0.16% for both M1 and M2, and 0.31% for both M3 and M4. For the 4 microsatellites, the Pearson's correlations were $R^2=0.99\%$ between the expected and observed MAFs, suggesting an accurate quantification of MSI alleles at a wide range of allelic frequencies.

2.3 Validation of multiplex drop-off ddPCR assays for MSI detection in MSI-H breast tumor DNA.

Following the validation of the drop-off ddPCR assays using cell line DNAs, the inventors performed MSI detection in 3 breast tumors (BC#1 to #3) previously known as MSI-H using a targeted NGS Panel (DRAGON) and IHC **(Figure 2)** by multiplex drop-off ddPCR assays.
As shown in **Figure 2,** all the 3 breast tumors were confirmed as MSI-H by drop-off ddPCR assays with at least 2 unstable microsatellites showing MAPs ranging from 1.29 to 25.3%. When analyzed using pentaplex PCR, only 2/3 samples (BC#2, BC#3) were identified as MSI-H. The remaining MSS sample (BC#1) displayed 5 stable microsatellites. These results prompted the inventors to carry out a blind analysis of MSI status in breast cancer by MSI-ddPCR assays.

2.4 Blind analysis and cross-validation of MSI status in BC samples

A blind analysis was then performed by drop-off ddPCR assays on 341 archived consecutive (FFPE) breast tumor DNAs with unknown MSI status (BC#4 to #344). Five (1.46%, 95%CI= [0.19%; 2.73%]) samples displayed at least 2 unstable microsatellites (MAFs values ranging from 1.35% to 12.58%) and were classified as MSI-H by ddPCR.
Among the 336 BC samples retrieved as MSS by ddPCR, 133 were submitted to targeted NGS panel: all were confirmed as MSS, demonstrating a 100% negative predictive value (95%CI= [97.74%; 100%]) of the present MSI-ddPCR assay.
The inventors then investigated the expression of MMR proteins (MLH1, MSH2, MSH6, PMS2) by IHC in the 5 breast tumors retrieved as MSI-H by the present assay. As shown in **Figure 3,** 3 tumors (BC#4, BC#6 and BC#8) displayed a loss of MLH1 and PMS2 proteins, while 2 (BC#5, BC#7) had an intact expression of MMR proteins. Pentaplex PCR analysis was also performed on these 5 samples: four were classified as MSS, one (BC#8) was MSI-H **(Figure 3).** These results substantiate that routine techniques to assess MSI status in colorectal or endometrial tumors, are not suited for BC.
The 5 breast tumors were further analyzed by a large targeted NGS panel. Four of them (BC#4, BC#6, BC#8 and BC#7, the latter classified as MSS by IHC staining) were classified as MSI-H with 5 or more microsatellite loci found unstable. However, BC#5 was classified as MSS, with only 2 unstable microsatellites out of 86 loci assessed by the large-panel NGS.
Finally, to investigate the possible molecular mechanisms responsible for MSI-H in these breast tumors, the inventors searched for the mutations in MMR genes or MLH1 promotor methylation. These analyses retrieved a homozygous deletion of MLH1 in BC#4, a homozygous deletion of MSH2 in BC#7, and a hypermethylation of MLH1 promoter in BC#6 and BC#8 **(Figure 3).** BC#5 showed no MMR mutation. MLH1 promoter methylation was not investigated in this tumor as MLH1 protein expression was conserved.

2.5 MSI detection in tumor DNA from tumor-infiltrating lymphocytes (TILs)-rich breast cancer patients

It has been reported that tumors with dMMR/MSI-H phenotype exhibit more tumor-infiltrating lymphocytes (TILs) than those with pMMR/MSS, in line with their better response to ICI (Fridman WH, et al. Nat Rev Cancer. Nature Publishing Group; 2012;12:298-306). The inventors therefore investigated the MSI prevalence in a series of TILs-rich BC (formerly known as breast medullary carcinomas): 11 cases with unknown MSI-H status were screened with the present MSI-ddPCR assay. As shown in **Figure 4,** one sample (TIL-BC#1) was detected as MSI-H, all 4 microsatellites being unstable (MAFs ranging from 0.61 to 8.74 %). The MSI-H status was also confirmed by targeted NGS panel, IHC, and pentaplex PCR **(Figure 4).** The targeted NGS panel did not retrieve any mutation in MLH1, MSH2, MSH6 or PMS2, and MLH1 promotor was not hypermethylated. Whole exome sequencing revealed a high CpG>TpG mutation rate and pathogenic frameshift variant in MSH3 that might be responsible for the observed MSI status.

2.6 MSI detection in circulating tumor DNA

The above results demonstrated the high sensitivity and specificity of these drop-off ddPCR assays for MSI detection in DNA from tissue. The inventors thus evaluated the assays' abilities for detecting MSI status from cfcDNA. As no plasma sample was available from the patients with MSI-H described above, plasmas from a new cohort of BC (27 out of 28 samples were metastatic) with previously unknown MSI status were then analyzed. One sample (P#1: metastatic BC) was detected as MSI-H with MAFs ranging from 8.23 to 49.69% **(Figure 5),** and the MSI-H status was confirmed by targeted NGS panel (MSI-H, n=1; MSS, n=27). This patient was later diagnosed with a Lynch syndrome harboring a germline MLH1 mutation. These results suggested the feasibility of using this method for non-invasive MSI detection in breast cancer patients.

2.7 MSI follow-up using cfcDNA from metastatic breast cancer (MBC) patient with MSI-H tumor treated with pembrolizumab

One MBC patient (P#29) was newly identified with MSI-H tumor by NGS panel which was confirmed by IHC, pentaplex PCR, and MSI-ddPCR assays **(Figure 6).** This patient, diagnosed with a metastatic triple negative BC experienced a symptomatic disease progression under a second line chemotherapy with carboplatin-gemcitabine and intrathecal methotrexate (for meningeal carcinomatosis). Following the characterization of her MSI status, this patient received pembrolizumab, an anti-PD-1 monoclonal antibody, as third line therapy.

To monitor treatment efficacy, plasma was collected before (baseline: B) and during pembrolizumab-based therapy (D5, D7, D11). MSI detection in cfcDNA was then performed with the use of MSI-ddPCR assays. As shown in **Figure 6A,** all the 4 microsatellites were found to be unstable (MAFs above the defined cut-offs) in plasma before pembrolizumab treatment started. After the first injection of pembrolizumab, an overall decrease of microsatellite MAFs was clearly observed at D5, D7 and D11 where 2/4 (M1, M3) unstable microsatellites were not anymore detected. The later-observed clinical improvement of the patient general condition confirmed the clinical response to pembrolizumab. Moreover, the same MAFs decrease was observed when analyzing PIK3CA E545K **(Figure 6B),** a mutation that was also identified in the patient tumor. These results suggest that MSI-ddPCR assays provide accurate and reliable ctDNA quantification as well as an overview of treatment response.

**Discussion**

There is no recommended method to screen MSI in BC patients. The absence of stereotyped pattern of MSI across tumor types implies that MSI detection could be customized, based on cancer type. In a study based on whole exome sequencing, cancer-specific microsatellite panels of 1 to 7 loci were needed to attain ≥95% diagnostic sensitivity and specificity for MSI detection in 11 non breast cancer types. Breast cancer was among the most difficult type of cancer to assess for MSI, as 800 microsatellite loci were needed to achieve comparable performances (Long DR, et al. Clin Chem. 2020;66:1310-8). The relevance of common methods such as IHC and pentaplex PCR, originally optimized to detect MSI in colorectal and endometrial cancers, is very questionable in breast cancer. While large NGS panel could interrogate dozens of microsatellites, their overall relevance for metastatic breast cancer care remains debated, with recent guidelines advocating against their use in routine practice (Mosele F, et al. Ann Oncol. 2020;31:1491-505). Taken together, the current MSI screening in breast cancer patients could be seen as mostly inexistant or inefficient, despite that the absolute number of BC patients with MSI-H tumors makes this population non-negligible. In that context, the drop-off ddPCR assays reported here are compatible with large scale screening at a limited cost and with short turnaround time.

Compared to two conventional methods, IHC and pentaplex PCR, the new drop-off ddPCR assays reported here showed clearly higher sensitivity for MSI detection in BC patients. The 4 microsatellites interrogated in the present assay were selected using whole genome sequencing data and are positioned at intergenic or intronic regions, i.e., not part of exonic microsatellites assessed by Long and colleagues (Long DR, et al. Clin Chem. 2020;66:1310-8).

The limited sensitivity of pentaplex PCR in breast cancer is illustrated by the missed detection of all the MSI-H tumors in the present study but also by the inconsistency of the detection of 1 MSI-H tumor within a series of 11 breast invasive carcinoma with medullary patterns (formerly known as medullary carcinoma), a subgroup where prior reports failed to identify MSI-H tumors by pentaplex PCR (Schmitt FC, et al. Int J Cancer. 1999;82:644-7; Lee S-C, et al. Am Soc Clin Pathol. 2001;115:823-7; Horimoto Y, et al. Cancer Sci. 2020;111:2647-54). In the present study, one tumor was detected as MSI-H in such subtype by ddPCR and confirmed by NGS, with a pathogenic frameshift variant in MSH3.

The second most commonly used technique to assess MSI status, MMR proteins IHC has already been reported as lacking sensitivity in breast cancer patients, mostly due to intra-tumor heterogeneity (Fusco N, et al. JNCI Cancer Spectr. 2018;2:4-6). In keeping with these results, the inventors found two tumors in which MMR protein IHC was intact, as missense mutations in MMR genes could yield to antigenically intact but non-functional proteins (Hechtman JF, et al. 2020;33:871-9 ; Gilson P, et al. Cancers. 2021;13).

Finally, using a large-scale multigene NGS panel as reference, the inventors observed a good concordance in MSI status detection between the present assay and the NGS panel, which interrogated 86 microsatellites. One hundred thirty-three breast cancer patients classified as MSS by drop-off ddPCR assays were also identified as MSS by targeted NGS panels. Four patients were identified as MSI-H by ddPCR assays and targeted NGS panel. A discrepancy, however, was observed for the 5th patient, being MSI-H by ddPCR but MSS by NGS panel and IHC. For that specific case, the low tumor mutational burden observed suggests a microsatellite-stable status, and therefore a false-positive of the present ddPCR approach (among a total of 380 tested cases). Overall, the present technique showed a 99% specificity, with one false positive result among all the NGS-panel confirmed MSS. Due to the inherent low incidence of MSI-H in breast cancer and the lack of a larger cohort with confirmed MSI-H samples, the present study cannot conclude on the assay sensitivity. Nevertheless, with 6 confirmed MSI-H cases, the incidence of MSI-H of BC (1.6%) reported here is in keeping with those described by prior reports by whole exome sequencing (WES) and WGS data analysis (1.7%), suggesting a good sensitivity of the present ddPCR assay for the detection of MSI in BC samples.

Recently, other NGS-based approaches have been developed for sensitive MSI detection in tumor tissue DNA (Hempelmann JA, et al. J Mol Diagnostics]. American Society for Investigative Pathology and the Association for Molecular Pathology; 2015;17:705-14;

Zhu L, et al. J Mol Diagnostics [Internet]. American Society for Investigative Pathology and the Association for Molecular Pathology; Gallon R, et al. Hum Mutat. 2020;41:332-41) or circulating tumor DNA (e.g. Guardant360® CDx (Guardant Health. Guardant360® CDx First FDA-Approved Liquid Biopsy for Comprehensive Tumor Mutation Profiling Across All Solid Cancers [Internet]. 2020), FoundationOne® Liquid CDx (Woodhouse R, et al. PLoS One. 2020;15:1-18), Georgiadis (Georgiadis A, et al. Clin Cancer Res. 2019;25:7024-34) showing a low limit of detection (0.09% of ctDNA content, Guardant360® CDx). However, NGS based assay are still costly and time consuming compared to MSI ddPCR assays. The inventors therefore propose a two-steps MSI detection strategy: firstly, performing a large-scale screening of breast tumors using the present newly developed drop-off ddPCR assays, for a fast, cost-effective and sensitive identification of cases with likely MSI-H status, and then confirm MSI-H status by an orthogonal technique, preferably NGS. This approach could significantly decrease the cost and shorten the time in obtaining the results, which corresponds well to the requirements for clinical application.

Beyond the quantification of MSI alleles and the identification of potential responders BC patients to immunotherapy, MSI-ddPCR is suitable for non-invasive monitoring of treatment efficacy and patient follow-up which could considerably improve patient management.

In conclusion, here in this study the inventors propose 4 newly identified MSI biomarkers combined with highly sensitive and specific drop-off ddPCR assays for MSI status identification in breast cancer. This method is compatible with tumor tissue DNA and cfcDNA and has the potential to be routinely applied in clinics, which could ultimately increase the access of the concerned patients to ICI therapy.

### Example 2

#### 1. Clinical samples:

Archived FFPE tissue specimens from colorectal cancer (n= 14) and endometrial cancer (n= 7) patients were retrieved from the Institut Curie Pathology and Genetics units. All the samples were previously classified as MSI-H by the pentaplex test.

#### 2. Results:

To evaluate the ddPCR assays ability to detect MSI-H in other tumor types, the inventors screened a collection of 21 DNA from FFPE tumor tissue samples (colorectal cancer, n= 14 ; endometrial cancer, n= 7) previously characterized as MSI-H using the pentaplex test. Considering a sample as MSI-H when at least two microsatellites were identified as unstable (MAF< defined cut-off value), all samples (n= 14) from colorectal cancer were found to be MSI-H by drop-off ddPCR assays, showing a 100% sensitivity. For endometrial cancer, 6 out of 7 samples were classified as MSI-H with the remaining MSS sample displaying MAF value above the cut-off value for 1 of the 4 microsatellites **(Figure 7).**

The MSI status retrieved by ddPCR assays is highly concordant with that reported by the pentaplex test for 20/21 samples (95%) suggesting that MSI ddPCR assays could be extended to identify MSI status in other tumor types.

**Claims**

1. An *in vitro* method for identifying a cancer patient presenting microsatellite unstable tumors, who is likely to benefit from immunotherapy, said method comprising the steps of:

   i) detecting a mutation within at least one microsatellite sequence in a plurality of nucleic acid molecules of a tumor DNA sample by subjecting said sample to a digital polymerase chain reaction, wherein said microsatellite sequence is selected from the group consisting of: M1 localized between positions 93963773 to 93963781 of chromosome 3 (GRch37.p13 : GCF_000001405.25, updated on 28/06/2013), M2 localized between positions 36126599 to 36126607 of chromosome 4 (GRch37.p13 : GCF_000001405.25, updated on 28/06/2013), M3 localized between positions 65403357 to 6543365 of chromosome 1 (GRch37.p13 : GCF_000001405.25, updated on 28/06/2013) and M4 localized between positions 178753846 to 1787538554 of chromosome 1 (GRch37.p13 : GCF_000001405.25, updated on 28/06/2013),
   ii) determining a copy number of each mutated microsatellite sequence(s) in said tumor DNA sample,

   wherein a higher copy number of at least one mutated microsatellite sequence(s) as compared to corresponding threshold value(s) is indicative that the patient presents microsatellite unstable tumors and is likely to benefit from immunotherapy.

2. The method according to claim 1 further comprising the steps of:

   iii) determining a copy number of each corresponding wild-type microsatellite sequence(s) in said tumor DNA sample and,
   iv) calculating for each microsatellite sequence, a mutant allelic frequency (MAF) of said microsatellite sequence with the following formula: MAF = copy number of mutated microsatellite sequence / (copy number of wild-type microsatellite sequence + copy number of mutated microsatellite sequence), wherein a higher MAF of at least one microsatellite sequence(s) as compared to a corresponding threshold value is indicative that the patient presents microsatellite unstable tumors and is likely to benefit from the immunotherapy.

3. The method of claim 1 or 2 wherein the mutation is detected within at least two microsatellite sequences selected from the group consisting of: M1, M2, M3 and M4, and wherein a higher copy number or MAF of at least two mutated microsatellite sequences as compared to corresponding threshold values is indicative that the patient presents microsatellite unstable tumors and is likely to benefit from the immunotherapy.

4. The method according to any one of claims 1 to 3 wherein said mutation is a deletion, a substitution and/or an addition of at least one repeat unit, preferably a deletion of at least one repeat unit.

5. The method according to any one of claims 1 to 4 wherein said digital polymerase chain reaction is a droplet digital polymerase chain reaction.

6. The method according to any one of claim 1 to 5 wherein said digital polymerase chain reaction comprises subjecting the tumor DNA sample to:

   - a pair of primers for amplifying a target sequence including a mutated or wild-type microsatellite sequence selected from the group consisting of M1, M2, M3 and M4,
   - an oligonucleotide probe (MS) comprising a sequence complementary to the wild-type microsatellite sequence and,
   - an oligonucleotide reference probe (REF) comprising a sequence complementary to a part of said target sequence located outside of said microsatellite sequence, preferably wherein said oligonucleotide microsatellite probe (MS) and oligonucleotide reference probe (REF) are labeled with different fluorophores.

7. The method according to any one of claims 1 to 6 wherein when said microsatellite sequence is M1, the pair of primers are SEQ ID NO: 1 and 2, the oligonucleotide microsatellite probe (MS) is SEQ ID NO: 3 and the oligonucleotide reference probe (REF) is SEQ ID NO: 4.

8. The method according to any one of claims 1 to 7 wherein when said microsatellite sequence is M2, the pair of primers are SEQ ID NO: 5 and 6, the oligonucleotide microsatellite probe (MS) is SEQ ID NO: 7 and the oligonucleotide reference probe (REF) is SEQ ID NO: 8.

9. The method according to any one of claims 1 to 8 wherein when said microsatellite sequence is M3, the pair of primers are SEQ ID NO: 9 and 10, the oligonucleotide microsatellite probe (MS) is SEQ ID NO: 11 and the oligonucleotide reference probe (REF) is SEQ ID NO: 12.

10. The method according to any one of claims 1 to 9 wherein when said microsatellite sequence is M4, the pair of primers are SEQ ID NO: 13 and 14, the oligonucleotide microsatellite probe (MS) is SEQ ID NO: 15 and the oligonucleotide reference probe (REF) is SEQ ID NO: 16.

11. The method according to any one of claims 1 to 10 wherein said tumor DNA sample is tumor tissue or cell-free circulating DNA in biological fluids, preferably in plasma.

12. The method according to any one of claims 1 to 11 wherein said cancer is selected from the group consisting of: lung cancer, prostate cancer, endometrial cancer, colorectal cancer, ovarian cancer, tumor of the Lynch syndrome, breast cancer and gastric cancer, preferably breast cancer.

13. An immunotherapy for use in the treatment of a cancer in a patient in need thereof wherein said immunotherapy is administered in a patient previously identified as presenting microsatellite unstable tumors and who is likely to benefit from an immunotherapy using the method according to any one of claims 1 to 12.

14. A method for evaluating the therapeutic response of an immunotherapy in a patient having a cancer, said method comprising:

   i) detecting a mutation within at least one microsatellite sequence in a plurality of nucleic acid molecules of a tumor DNA sample of a patient having received at least one dose of an immunotherapy, by subjecting said sample to a digital polymerase chain reaction, wherein said microsatellite sequence is selected from the group consisting of: M1, M2, M3 and M4,

   wherein a decrease of copy number or MAF of at least one mutated microsatellite sequence in the patient sample during the treatment is indicative that the patient is responsive to the immunotherapy.

15. A kit for identifying a mutation in a microsatellite sequence selected from the group consisting of: M1, M2, M3 and M4 in a DNA sample comprising:

   - a pair of primers for amplifying a target sequence including a mutated or wild-type microsatellite sequence selected from the group consisting of M1, M2, M3 and M4,
   - an oligonucleotide probe (MS) comprising a sequence complementary to the wild-type microsatellite sequence and,
   - an oligonucleotide reference probe (REF) comprising a sequence complementary to a part of said target sequence located outside of said microsatellite sequence, preferably

      - a pair of primers of SEQ ID NO: 1 and 2, the oligonucleotide microsatellite probe (MS) of SEQ ID NO: 3 and the oligonucleotide reference probe (REF) of SEQ ID NO: 4,
      - a pair of primers of SEQ ID NO: 5 and 6, the oligonucleotide microsatellite probe (MS) of SEQ ID NO: 7 and the oligonucleotide reference probe (REF) of SEQ ID NO: 8,
      - a pair of primers of SEQ ID NO: 9 and 10, the oligonucleotide microsatellite probe (MS) of SEQ ID NO: 11 and the oligonucleotide reference probe (REF) of SEQ ID NO: 12 and/or
      - a pair of primers of SEQ ID NO: 13 and 14, the oligonucleotide microsatellite probe (MS) of SEQ ID NO: 15 and the oligonucleotide reference probe (REF) of SEQ ID NO: 16.

**Patentansprüche**

1. *In-vitro-Verfahren* zum Identifizieren eines Krebspatienten, welcher Mikrosatelliten-instabile Tumore aufweist und wahrscheinlich von einer Immuntherapie profitieren wird, wobei das Verfahren die Schritte umfasst aus:

   i) Nachweisen einer Mutation innerhalb mindestens einer Mikrosatellitensequenz in einer Mehrzahl von Nukleinsäuremolekülen einer Tumor-DNA-Probe durch Unterziehen der Probe einer digitalen Polymerasekettenreaktion, wobei die Mikrosatellitensequenz ausgewählt ist aus der Gruppe bestehend aus: M1, welche zwischen Positionen 93963773 bis 93963781 auf Chromosom 3 lokalisiert ist (GRch37.p13 : GCF_000001405.25, aktualisiert am 28.06.2013), M2, welche zwischen Positionen 36126599 bis 36126607 auf Chromosom 4 lokalisiert ist (GRch37.p13 : GCF_000001405.25, aktualisiert am 28.06.2013), M3, welche zwischen Positionen 65403357 bis 6543365 auf Chromosom 1 lokalisiert ist (GRch37.p13 : GCF_000001405.25, aktualisiert am 28.06.2013) und M4, welche zwischen Positionen 178753846 bis 1787538554 auf Chromosom 1 lokalisiert ist (GRch37.p13 : GCF_000001405.25, aktualisiert am 28.06.2013),
   ii) Bestimmen einer Kopienzahl von jeder mutierten Mikrosatellitensequenz(en) in der Tumor-DNA-Probe,

   wobei eine höhere Kopienzahl von mindestens einer mutierter Mikrosatellitensequenz(en) im Vergleich mit entsprechenden Schwellenwert(en) anzeigt, dass der Patient Mikrosatelliten-instabile Tumore aufweist und wahrscheinlich von einer Immuntherapie profitieren wird.

2. Verfahren nach Anspruch 1, ferner umfassend die Schritte aus:

   iii) Bestimmen einer Kopienzahl von jeder entsprechendedn Wildtyp-Mikrosatellitensequenz(en) in der Tumor-DNA-Probe und,
   iv) Berechnen für jede Mikrosatellitensequenz einer mutierten allelischen Frequenz (MAF) der Mikrosatellitensequenz mit der folgenden Formel: MAF = Kopienzahl der mutierten Mikrosatellitensequenz / (Kopienzahl der Wildtyp-Mikrosatellitensequenz + Kopienzahl der mutierten Mikrosatellitensequenz), wobei eine höhere MAF mindestens einer Mikrosatellitensequenz im Vergleich zu einem entsprechenden Schwellenwert darauf hinweist, dass der Patient Mikrosatelliten-instabile Tumore aufweist und wahrscheinlich von der Immuntherapie profitieren wird.

3. Verfahren nach Anspruch 1 oder 2, wobei die Mutation innerhalb von mindestens zwei Mikrosatellitensequenzen nachgewiesen wird, welche ausgewählt sind aus der Gruppe bestehend aus: M1, M2, M3 und M4, und wobei eine höhere Kopienzahl oder MAF von mindestens zwei mutierten Mikrosatellitensequenzen im Vergleich zu entsprechenden Schwellenwerten darauf hinweist, dass der Patient Mikrosatelliten-instabile Tumore aufweist und wahrscheinlich von der Immuntherapie profitieren wird.

4. Verfahren nach einem der Ansprüche 1 bis 3, wobei die Mutation eine Deletion, eine Substitution und/oder eine Addition von mindestens einer Wiederholungseinheit ist, vorzugsweise eine Deletion von mindestens einer Wiederholungseinheit.

5. Verfahren nach einem der Ansprüche 1 bis 4, wobei die digitale Polymerasekettenreaktion eine digitale Tropfen-Polymerasekettenreaktion ist.

6. Verfahren nach einem der Ansprüche 1 bis 5, wobei die digitale Polymerasekettenreaktion umfasst, dass die Tumor-DNA-Probe unterzogen wird:

   - einem Paar von Primern zum Amplifizieren einer Zielsequenz, umfassend eine mutierte oder Wildtyp-Mikrosatellitensequenz, ausgewählt aus der Gruppe bestehend aus M1, M2, M3 und M4,
   - einer Oligonukleotid-Sonde (MS), umfassend eine Sequenz, welche zu der Wildtyp-Mikrosatellitensequenz komplementär ist, und
   - eine Oligonukleotid-Referenzsonde (REF), welche eine Sequenz umfasst, welche zu einem Teil der Zielsequenz komplementär ist, welcher sich außerhalb der Mikrosatellitensequenz befindet, vorzugsweise wobei die Oligonukleotid-Mikrosatelliten-Sonde (MS) und die Oligonukleotid-Referenzsonde (REF) mit unterschiedlichen Fluorophoren markiert sind.

7. Verfahren nach einem der Ansprüche 1 bis 6, wobei, wenn die Mikrosatellitensequenz M1 ist, das Paar von Primern SEQ ID NO: 1 und 2 sind, die Oligonukleotid-Mikrosatelliten-Sonde (MS) SEQ ID NO: 3 ist und die Oligonukleotid-Referenzsonde (REF) SEQ ID NO: 4 ist.

8. Verfahren nach einem der Ansprüche 1 bis 7, wobei, wenn die Mikrosatellitensequenz M2 ist, das Paar von Primern SEQ ID NO: 5 und 6 sind, die Oligonukleotid-Mikrosatelliten-Sonde (MS) SEQ ID NO: 7 ist und die Oligonukleotid-Referenzsonde (REF) SEQ ID NO: 8 ist.

9. Verfahren nach einem der Ansprüche 1 bis 8, wobei, wenn die Mikrosatellitensequenz M3 ist, das Paar von Primern SEQ ID NO: 9 und 10 sind, die Oligonukleotid-Mikrosatelliten-Sonde (MS) SEQ ID NO: 11 ist und die Oligonukleotid-Referenzsonde (REF) SEQ ID NO: 12 ist.

10. Verfahren nach einem der Ansprüche 1 bis 9, wobei, wenn die Mikrosatellitensequenz M4 ist, das Paar von Primern SEQ ID NO: 13 und 14 sind, die Oligonukleotid-Mikrosatelliten-Sonde (MS) SEQ ID NO: 15 ist und die Oligonukleotid-Referenzsonde (REF) SEQ ID NO: 16 ist.

11. Verfahren nach einem der Ansprüche 1 bis 10, wobei die Tumor-DNA-Probe Tumorgewebe oder zellfreie zirkulierende DNA in biologischen Fluiden ist, vorzugsweise in Plasma.

12. Verfahren nach einem der Ansprüche 1 bis 11, wobei der Krebs ausgewählt ist aus der Gruppe bestehend aus: Lungenkrebs, Prostatakrebs, Gebärmutterkrebs, kolorektalem Krebs, Eierstockkrebs, Tumor des Lynch-Syndroms, Brustkrebs und Magenkrebs, vorzugsweise Brustkrebs.

13. Immuntherapie zur Verwendung bei der Behandlung von einem Krebs bei einem Patienten, welcher dessen bedarf, wobei die Immuntherapie einem Patienten verabreicht wird, bei welchem zuvor identifiziert wurde, dass dieser Mikrosatelliten-instabile Tumore aufweist und welcher wahrscheinlich von einer Immuntherapie unter Verwendung des Verfahrens nach einem der Ansprüche 1 bis 12 profitieren wird.

14. Verfahren zum Bewerten des therapeutischen Ansprechens einer Immuntherapie bei einem Patienten, welcher an einem Krebs erkrankt ist, wobei das Verfahren umfasst:

i) Nachweisen einer Mutation innerhalb mindestens einer Mikrosatellitensequenz in einer Mehrzahl von Nukleinsäuremolekülen einer Tumor-DNA-Probe eines Patienten, welcher mindestens eine Dosis einer Immuntherapie erhalten hat, indem die Probe einer digitalen Polymerasekettenreaktion unterzogen wird, wobei die Mikrosatellitensequenz ausgewählt ist aus der Gruppe bestehend aus: M1, M2, M3 und M4, wobei eine Abnahme der Kopienzahl oder MAF mindestens einer mutierten Mikrosatellitensequenz in der Patientenprobe während der Behandlung darauf hinweist, dass der Patient auf die Immuntherapie anspricht.

15. Kit zum Identifizieren einer Mutation in einer Mikrosatellitensequenz, ausgewählt aus der Gruppe bestehend aus: M1, M2, M3 und M4 in einer DNA-Probe, umfassend:

- ein Paar von Primern zum Amplifizieren einer Zielsequenz, umfassend eine mutierte oder Wildtyp-Mikrosatellitensequenz, ausgewählt aus der Gruppe bestehend aus M1, M2, M3 und M4,
- eine Oligonukleotid-Sonde (MS), umfassend eine Sequenz, welche zu der Wildtyp-Mikrosatellitensequenz komplementär ist, und
- eine Oligonukleotid-Referenzsonde (REF), umfassend eine Sequenz, welche zu einem Teil der Zielsequenz komplementär ist, welcher sich außerhalb der Mikrosatellitensequenz befindet, vorzugsweise

- ein Paar von Primern von SEQ ID NO: 1 und 2, die Oligonukleotid-Mikrosatelliten-Sonde (MS) von SEQ ID NO: 3 und die Oligonukleotid-Referenzsonde (REF) von SEQ ID NO: 4,
- ein Paar von Primern von SEQ ID NO: 5 und 6, die Oligonukleotid-Mikrosatelliten-Sonde (MS) von SEQ ID NO: 7 und die Oligonukleotid-Referenzsonde (REF) von SEQ ID NO: 8,
- ein Paar von Primern von SEQ ID NO: 9 und 10, die Oligonukleotid-Mikrosatelliten-Sonde (MS) von SEQ ID NO: 11 und die Oligonukleotid-Referenzsonde (REF) von SEQ ID NO: 12 und/oder
- ein Paar von Primern von SEQ ID NO: 13 und 14, die Oligonukleotid-Mikrosatelliten-Sonde (MS) von SEQ ID NO: 15 und die Oligonukleotid-Referenzsonde (REF) von SEQ ID NO: 16.

**Revendications**

1. Procédé *in vitro* d'identification d'un patient cancéreux présentant des tumeurs à instabilité microsatellitaire, qui est susceptible de bénéficier d'une immunothérapie, ledit procédé comprenant les étapes suivantes :

   i) la détection d'une mutation au sein d'au moins une séquence microsatellite dans une pluralité de molécules d'acide nucléique d'un échantillon d'ADN tumoral en soumettant ledit échantillon à une amplification en chaîne par polymérase numérique, dans lequel ladite séquence microsatellite est sélectionnée dans le groupe constitué de : M1 localisé entre les positions 93963773 à 93963781 du chromosome 3 (GRch37.p13 : GCF_000001405.25, mis à jour le 28/06/2013), M2 localisé entre les positions 36126599 à 36126607 du chromosome 4 (GRch37.p13 : GCF_000001405.25, mis à jour le 28/06/2013), M3 localisé entre les positions 65403357 à 6543365 du chromosome 1 (GRch37.p13 : GCF_000001405.25, mis à jour le 28/06/2013) et M4 localisé entre les positions 178753846 à 1787538554 du chromosome 1 (GRch37.p13 : GCF_000001405.25, mis à jour le 28/06/2013),
   ii) la détermination d'un nombre de copies de chaque séquence microsatellite mutée dans ledit échantillon d'ADN tumoral,

   dans lequel un nombre de copies d'au moins une séquence microsatellite mutée supérieur à une valeur seuil correspondante indique que le patient présente des tumeurs à instabilité microsatellitaire et est susceptible de bénéficier d'une immunothérapie.

2. Procédé selon la revendication 1, comprenant en outre les étapes suivantes :

   iii) la détermination d'un nombre de copies de chacune des séquences microsatellites de type sauvage correspondantes dans ledit échantillon d'ADN tumoral, et
   iv) le calcul pour chaque séquence microsatellite, d'une fréquence allélique mutante (MAF) de ladite séquence microsatellite avec la formule suivante : MAF = nombre de copies de séquence microsatellite mutée/(nombre de copies de séquence microsatellite de type sauvage + nombre de copies de séquence microsatellite mutée), dans lequel une MAF d'au moins une séquence microsatellite supérieure à une valeur seuil correspondante indique que le patient présente des tumeurs à instabilité microsatellitaire et est susceptible de bénéficier de l'immunothérapie.

3. Procédé selon la revendication 1 ou 2, dans lequel la mutation est détectée au sein d'au moins deux séquences microsatellites sélectionnées dans le groupe constitué de : M1, M2, M3 et M4, et dans lequel un nombre de copies ou une MAF d'au moins deux séquences microsatellites mutées supérieur(e) à des valeurs seuils correspondantes indique que le patient présente des tumeurs instables de microsatellites et est susceptible de bénéficier de l'immunothérapie.

4. Procédé selon l'une quelconque des revendications 1 à 3, dans lequel ladite mutation est une délétion, une substitution et/ou une addition d'au moins un motif répétitif, de préférence une délétion d'au moins un motif répétitif.

5. Procédé selon l'une quelconque des revendications 1 à 4, dans lequel ladite amplification en chaîne par polymérase numérique est une amplification en chaîne par polymérase numérique en gouttelettes.

6. Procédé selon l'une quelconque des revendications 1 à 5, dans lequel ladite amplification en chaîne par polymérase numérique comprend la soumission de l'échantillon d'ADN tumoral à :

   - une paire d'amorces pour amplifier une séquence cible comprenant une séquence microsatellite mutée ou de type sauvage sélectionnée dans le groupe constitué de M1, M2, M3 et M4,
   - une sonde oligonucléotide (MS) comprenant une séquence complémentaire à la séquence microsatellite de type sauvage et,
   - une sonde oligonucléotidique de référence (REF) comprenant une séquence complémentaire à une partie de ladite séquence cible située hors de ladite séquence microsatellite, dans laquelle ladite sonde oligonucléotidique microsatellite (MS) et ladite sonde oligonucléotidique de référence (REF) sont marquées avec des fluorophores différents.

7. Procédé selon l'une quelconque des revendications 1 à 6 dans lequel lorsque ladite séquence microsatellite est M1, la paire d'amorces est SEQ ID NO : 1 et 2, la sonde oligonucléotidique microsatellite (MS) est SEQ ID NO: 3 et la sonde oligonucléotidique de référence (REF) est SEQ ID NO : 4.

8. Procédé selon l'une quelconque des revendications 1 à 7 dans lequel lorsque ladite séquence microsatellite est M2, la paire d'amorces est SEQ ID NO : 5 et 6, la sonde oligonucléotidique microsatellite (MS) est SEQ ID NO: 7 et la sonde oligonucléotidique de référence (REF) est SEQ ID NO : 8.

9. Procédé selon l'une quelconque des revendications 1 à 8, dans lequel lorsque ladite séquence microsatellite est M3, la paire d'amorces est SEQ ID NO : 9 et 10, la sonde oligonucléotidique microsatellite (MS) est SEQ ID NO:11 et la sonde oligonucléotidique de référence (REF) est SEQ ID NO : 12.

10. Procédé selon l'une quelconque des revendications 1 à 9, dans lequel lorsque ladite séquence microsatellite est M4, la paire d'amorces est SEQ ID NO : 13 et 14, la sonde oligonucléotidique microsatellite (MS) est SEQ ID NO : 15 et la sonde oligonucléotidique de référence (REF) est SEQ ID NO : 16.

11. Procédé selon l'une quelconque des revendications 1 à 10, dans lequel ledit échantillon d'ADN tumoral est un tissu tumoral ou un ADN circulant acellulaire dans des fluides biologiques, de préférence dans du plasma.

12. Procédé selon l'une quelconque des revendications 1 à 11, dans lequel ledit cancer est sélectionné dans le groupe constitué de : cancer du poumon, cancer de la prostate, cancer de l'endomètre, cancer colorectal, cancer de l'ovaire, tumeur du syndrome de Lynch, cancer du sein et cancer gastrique, de préférence cancer du sein.

13. Immunothérapie destinée à être utilisée dans le traitement d'un cancer chez un patient qui en a besoin, dans laquelle ladite immunothérapie est administrée à un patient précédemment identifié comme présentant des tumeurs à instabilité microsatellitaire et qui est susceptible de bénéficier d'une immunothérapie en utilisant le procédé selon l'une quelconque des revendications 1 à 12.

14. Procédé d'évaluation de la réponse thérapeutique d'une immunothérapie chez un patient atteint d'un cancer, ledit procédé comprenant :

i) la détection d'une mutation au sein d'au moins une séquence microsatellite dans une pluralité de molécules d'acide nucléique d'un échantillon d'ADN tumoral d'un patient ayant reçu au moins une dose d'une immunothérapie, en soumettant ledit échantillon à une amplification en chaîne par polymérase numérique, dans lequel ladite séquence microsatellite est sélectionnée dans le groupe constitué de : M1, M2, M3 et M4, dans lequel une diminution du nombre de copies ou de la MAF d'au moins une séquence microsatellite mutée dans l'échantillon de patient pendant le traitement indique que le patient répond à l'immunothérapie.

15. Kit d'identification d'une mutation dans une séquence microsatellite sélectionnée dans le groupe constitué de : M1, M2, M3 et M4 dans un échantillon d'ADN comprenant :

- une paire d'amorces pour amplifier une séquence cible comprenant une séquence microsatellite mutée ou de type sauvage sélectionnée dans le groupe constitué de M1, M2, M3 et M4, - une sonde oligonucléotidique (MS) comprenant une séquence complémentaire à la séquence microsatellite de type sauvage, et
- une sonde oligonucléotidique de référence (REF) comprenant une séquence complémentaire à une partie de ladite séquence cible située hors de ladite séquence microsatellite, de préférence
- une paire d'amorces de SEQ ID NO : 1 et 2, la sonde oligonucléotidique microsatellite (MS) est SEQ ID NO : 3 et la sonde oligonucléotidique de référence (REF) est SEQ ID NO : 4,
- une paire d'amorces de SEQ ID NO : 5 et 6, la sonde oligonucléotidique microsatellite (MS) est SEQ ID NO : 7 et la sonde oligonucléotidique de référence (REF) est SEQ ID NO : 8,
- une paire d'amorces de SEQ ID NO : 9 et 10, la sonde oligonucléotidique microsatellite (MS) est SEQ ID NO : 11 et la sonde oligonucléotidique de référence (REF) est SEQ ID NO : 12 et/ou
- une paire d'amorces de SEQ ID NO : 13 et 14, la sonde oligonucléotidique microsatellite (MS) est SEQ ID NO : 15 et la sonde oligonucléotidique de référence (REF) est SEQ ID NO : 16.

**FIG. 1A-B**

**FIG. 1C**

EP 4 499 876 B1

**FIG. 1C (Continued)**

**Drop-off ddPCR**

FIG. 2A

## Drop-off ddPCR

FIG. 2A (cont.)

FIG. 2B

C

| BC samples | ddPCR | MMR IHC | NGS | | | MLH1 methylation | Pentaplex PCR |
|---|---|---|---|---|---|---|---|
| | | | MSI status | MMR gene alteration | TMB (variants/Mb) | | |
| BC#1 | MSI-H M3, M4 | Loss of MSH2 | MSI-H | Pathogenic mutation in MSH2 and LOH | 49.8 | Not analyzed | MSS |
| BC#2 | MSI-H M3, M4 | Loss of MSH2 & MSH6 | MSI-H | Pathogenic mutation in MSH2 and LOH | 34 | Not analyzed | MSI-H |
| BC#3 | MSI-H M1, M3, M4 | Loss of MLH1 | MSI-H | Not detected | 10.9 | MLH1 methylation | MSI-H |

**FIG. 2C**

FIG. 3A

EP 4 499 876 B1

**B**

| BC samples | ddPCR | MMR IHC | NGS | | | MLH1 methylation | Pentaplex PCR |
|---|---|---|---|---|---|---|---|
| | | | MSI status | MMR gene alteration | TMB (variants/MB) | | |
| BC#4 | MSI-H M1, M2, M3 | Loss of MLH1 & PMS2 | MSI-H | MLH1 homozygous deletion | 22.7 | Not analyzed | MSS |
| BC#5 | MSI-H M1, M2 | No loss | MSS | Not detected | 2 | Not analyzed | MSS |
| BC#6 | MSI-H M1, M3 | Loss of MLH1 & PMS2 | MSI-H | Not detected | 13.9 | MLH1 methylation | MSS |
| BC#7 | MSI-H M1, M2, M3, M4 | No loss | MSI-H | MSH2 homozygous deletion | 8 | Not analyzed | MSS |
| BC#8 | MSI-H M1, M3, M4 | Loss of MLH1 & PMS2 | MSI-H | Not detected | 12 | MLH1 methylation | MSI-H |

**FIG. 3B**

FIG. 4A-B

EP 4 499 876 B1

EP 4 499 876 B1

| BC sample | ddPCR | MMR IHC | NGS | | | Pentaplex PCR |
| | | | MSI status | MMR gene alteration | TMB (variants/MB) | |
|---|---|---|---|---|---|---|
| TIL-BC#1 | MSI-H M1, M3, M4 | Loss of MLH1 & PMS2 | MSI-H | MSH3 frameshift indel MLH3 frameshift indel MSH3 in-frame indel | 15 | MSI-H |

**FIG. 4C**

FIG. 5A-B

**FIG. 5C**

EP 4 499 876 B1

FIG. 6A-B

| | | | | NGS | | | |
|---|---|---|---|---|---|---|---|
| BC sample | ddPCR | MMR IHC | MSI status | MMR gene alteration | TMB (variants/Mb) | Pentaplex PCR |
| P#29 | MSI-H M1, M2, M3, M4 | Loss of MLH1 & PMS2 | MSI-H | MLH1 homozygous deletion | 15.9 | MSI-H |

**FIG. 6C**

**FIG. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021130271 A

**Non-patent literature cited in the description**

- **LORENZI M et al.** *J Oncol.*, 2020
- **CICEK MS et al.** *J Mol Diagnostics.*, 2011, vol. 13, 271-81
- **MCCONECHY MK et al.** *Gynecol Oncol.*, 2015, vol. 137, 306-10
- **HAUSE RJ et al.** *Nat Med.*, 2016, vol. 22, 1342-50
- **BRAHMER J et al.** *N Engl J Med.*, 2015, vol. 373, 123-35
- **BALAR A et al.** *Lancet.*, 2017, vol. 389, 67-76
- **LE DT ; DURHAM JN et al.** *Science*, 2017, vol. 357, 409-13
- **SURAWEERA N et al.** *Gastroenterology*, 2002, vol. 123, 1804-11
- **EIRIKSDOTTIR et al.** *INTERNATIONAL JOURNAL OF ONCOLOGY*, 1995
- **WILLIS J ; LEFTEROVA MI et al.** *Clin Cancer Res.*, 2019, vol. 25, 7035-45
- **BONNEVILLE R et al.** *JCO Precis Oncol.*, 2017, 1-15
- **CORTES-CIRIANO I et al.** *Nat Commun*, 2017, vol. 8, 1-12
- **MOSELE F et al.** *Ann Oncol.*, 2020, vol. 31, 1491-505
- **SAIKI et al.** *Science*, 1985, vol. 230, 1350-1354
- **SYKES et al.** Quantitation of targets for PCR by use of limiting dilution.. *BioTechniques*, 1992, vol. 13, 444-449
- **VOGELSTEIN ; KINZLER.** Digital PCR.. *Proc Natl Acad Sci USA*, 1999, vol. 96, 9236-9241
- **POHL ; SHIHLE.** Principle and applications of digital PCR.. *Expert Rev Mol Diagn*, 2004, vol. 4, 41-47
- **MONYA BAKER.** *Nature Methods*, 2012, vol. 9, 541-544
- **WARREN et al.** Transcription factor profiling in individual hematopoietic progenitors by digital RT-PCR. *Proc Natl Acad Sci USA*, 2006, vol. 103, 17807-17812
- **OTTESEN et al.** Microfluidic digital PCR enables multigene analysis of individual environmental bacteria. *Science*, 2006, vol. 314, 1464-1467
- **FAN ; QUAKE.** Detection of aneuploidy with digital polymerase chain reaction. *Anal Chem*, 2007, vol. 79, 7576-7579
- **MORRISON et al.** Nanoliter high-throughput quantitative PCR. *Nucleic Acids Res*, 2006, vol. 34, e123
- **SUNDBERG et al.** Spinning disk platform for microfluidic digital polymerase chain reaction. *Anal Chem*, 2010, vol. 82, 1546-1550
- **HINDSON ; BENJAMIN et al.** High-Throughput Droplet Digital PCR System for Absolute Quantitation of DNA Copy Number. *Analytical Chemistry*, 2011, vol. 83 (22), 8604-8610
- **HINSON et al.** *Anal. Chem.*, 2011, vol. 83, 8604-8610
- **PINHEIRO et al.** *Anal. Chem.*, 2012, vol. 84, 1003-1011
- **RONALD J HAUSE et al.** *Nat. Med*, 2016 (39)
- **LEON et al.** *Cancer Res*, 1977, vol. 37, 646-650
- **STROUN et al.** *Oncology*, 1989, vol. 46, 318-322
- **PARDOLL.** *Nature Rev Cancer*, 2012, vol. 12, 252-264
- **MELLMAN et al.** *Nature*, 2011, vol. 480, 480-489
- **NEWTON ; GRAHAM.** PCR, BIOS. Scientific Publishers, Ltd., 1994
- **SILVEIRA AB et al.** *Clin Chem.*, 2020, vol. 66, 606-13
- **DAVIES H et al.** *Cancer Res.*, 2017, vol. 77, 4755-62
- **FRIDMAN WH et al.** Nat Rev Cancer.. Nature Publishing Group, 2012, vol. 12, 298-306
- **LONG DR et al.** *Clin Chem.*, 2020, vol. 66, 1310-8
- **SCHMITT FC et al.** *Int J Cancer.*, 1999, vol. 82, 644-7
- **LEE S-C et al.** *Am Soc Clin Pathol.*, 2001, vol. 115, 823-7
- **HORIMOTO Y et al.** *Cancer Sci.*, 2020, vol. 111, 2647-54

- **FUSCO N et al.** *JNCI Cancer Spectr.*, 2018, vol. 2, 4-6
- **GILSON P et al.** *Cancers.*, 2021, 13
- **HEMPELMANN JA et al.** J Mol Diagnostics. American Society for Investigative Pathology and the Association for Molecular Pathology, 2015, vol. 17, 705-14
- **ZHU L et al.** J Mol Diagnostics [Internet. American Society for Investigative Pathology and the Association for Molecular Pathology
- **GALLON R et al.** *Hum Mutat.*, 2020, vol. 41, 332-41
- **WOODHOUSE R et al.** *PLoS One.*, 2020, vol. 15, 1-18
- **GEORGIADIS A et al.** *Clin Cancer Res.*, 2019, vol. 25, 7024-34